# EUROPEAN PATENT APPLICATION

(11) **EP 3 741 375 A1**
(43) Date of publication of application: **25.11.2020**
(21) Application number: 20166731.8
(22) Date of filing: 16.07.2015
(51) Int. Cl.: A61K 33/14, A61P 21/00, A61P 25/00, A61K 31/00, A61K 31/4166

(54) **METHODS FOR TREATING NEUROMUSCULAR JUNCTION-RELATED DISEASES**

(30) Priority: 17.07.2014 EP 14306159; 17.12.2014 EP 14307063
(62) Divisional of application: 15738353.0
(71) Applicant: INSERM (Institut National de la Santé et de la Recherche Médicale), 75013 Paris (FR); Centre National de la Recherche Scientifique, 75016 Paris (FR); UNIVERSITE PARIS DESCARTES, 75006 Paris (FR); Université Paris Diderot Paris 7, 75013 Paris (FR)
(72) Inventor: STROCHLIC, Laure, 75270 Paris cedex 06 (FR); MESSEANT, Julien, 75651 Paris cedex 13 (FR); DELERS, Perrine, 75270 Paris cedex 06 (FR); DOBBERTIN, Alexandre, 75270 Paris cedex 06 (FR); LEGAY, Claire, 75270 Paris cedex 06 (FR)
(74) Representative: Cabinet Becker et Associés

(57) **Abstract**

The invention relates to an inhibitor of glycogen synthase kinase 3 (GSK3) for use in treating a neuromuscular junction-related disease.

## Description

### FIELD OF THE INVENTION:

The present invention relates to methods for treating neuromuscular junction-related diseases.

### BACKGROUND OF THE INVENTION:

The neuromuscular junction (NMJ) is a cholinergic synapse between motor neurons and skeletal muscle fibers. The formation of this chemical synapse is based on the establishment of a trans-synaptic dialogue between presynaptic motor axons and postsynaptic muscle fibers (Sanes and Lichtman, 2001).

Altered neuromuscular transmission leads to a large number of diseases. Mutations in genes or autoantibodies directed against proteins critical for NMJ formation and maintenance are responsible for myasthenic syndromes (MS), heterogeneous disorders mainly characterized by varying degrees of skeletal muscles weakness and excessive fatigability (Berrih-Aknin et al., 2014; Eymard et al., 2013; Hantaï et al., 2013). The muscle-specific tyrosine kinase receptor MuSK and its co-receptor LRP4, a member of the low density lipoprotein receptor constitute the central hub regulating all steps of NMJ formation and maintenance (DeChiara et al., 1996; Hesser et al., 2006; Kim and Burden, 2008; Valenzuela et al., 1995; Weatherbee et al., 2006). As such, MuSK is a target for antibodies in the autoimmune disorder myasthenia gravis (MG) and several mutations both in its kinase and extracellular domains are responsible for *MUSK*-associated congenital MS (CMS) in human (Ben Ammar et al., 2013; Chevessier et al., 2004; Maselli et al., 2010; Mihaylova et al., 2009; Vincent and Leite, 2005). MuSK contains in its extracellular region a frizzled-like domain (cysteine-rich domain, CRD), known to interact with Wnt molecules (DeChiara et al., 1996; Masiakowski and Yancopoulos, 1998; Stiegler et al., 2009). Of particular interest for this study, MuSK CRD together with Ig1/2 autoantibodies were recently identified in anti-AChR (acetylcholine receptor) negative MG patients and one case of a severe CMS linked to homozygous deletion in MuSK ectodomain leading to the deletion of most of the CRD has been identified (Takamori, 2012; Takamori et al., 2013; Koenig et al., unpublished data).

NMJ formation relies upon the nerve-secreted agrin that binds to LRP4 and subsequently activates MuSK in cis. Agrin-induced MuSK activation stimulates multiple signaling pathways leading to the clustering and remodeling of AChR (Kim et al., 2008; Zhang et al., 2008, 2011). During the early steps of NMJ formation, before innervation, AChR clusters are aggregated in a broad central and prospective region of the muscle (Arber et al., 2002; Lin et al., 2001; Yang et al., 2001). This process, called muscle prepatterning, in which the muscle target gets ready to receive synaptic contacts is based on activation of the complex MuSK/Lrp4 (Jing et al., 2009; Kim and Burden, 2008). However, signaling mechanisms regulating AChR prepatterning remain largely unknown. Increasing evidence indicate a role of Wnt signaling in NMJ formation (Budnik and Salinas, 2011; Speese and Budnik, 2007; Wu et al., 2010). Wnt is a family of secreted glycoproteins involved in numerous developmental pathways, including synapse formation and axon guidance (van Amerongen and Nusse, 2009; Dickins and Salinas, 2013; Salinas, 2012; Willert and Nusse, 2012). Wnt proteins play also an essential role during skeletal muscle development and regeneration (von Maltzahn et al., 2012). At the NMJ, Wnt4 and Wnt11 are required for muscle prepatterning and axon guidance (Jing et al., 2009; Gordon et al., 2012; Strochlic et al., 2012). In addition, Wnt4, Wnt9a and Wnt11 are able to bind MuSK through its CRD (Strochlic et al., 2012; Zhang et al., 2012). Taken together, these data suggest that dysfunction of MuSK CRD can be associated with the onset of myasthenic syndromes in patients, however, the physiopathological mechanisms underlying the role of MuSK CRD in Wnt induced NMJ formation and maintenance remains to be explored.

### SUMMARY OF THE INVENTION:

The present invention relates to methods for treating neuromuscular junction-related diseases. In particular, the present invention is defined by the claims.

### DETAILED DESCRIPTION OF THE INVENTION:

Synapse formation relies upon the establishment of a trans-synaptic dialogue between pre- and postsynaptic cells. At the neuromuscular synapse (NMJ), perturbation of this critical dialogue leads to neuromuscular disorders such as myasthenic syndromes. The muscle-specific tyrosine kinase receptor MuSK contains in its extracellular region a frizzled-like domain (cysteine-rich domain, CRD), known to interact with Wnt molecules required for muscle prepatterning and axon guidance during NMJ formation. Dysfunction of MuSK CRD can be associated with the onset of myasthenic syndromes, however MuSK CRD function in Wnt induced NMJ formation and maintenance remains to be elucidated. Here, the inventors found that CRD deletion of MuSK in mice caused strong defects of both muscle prepatterning and synapse differentiation including (i) a drastic deficit in AChR clusters number, size and density and (ii) excessive motor axons growth that bypass AChR clusters. NMJs are able to form and mutant mice are viable, but progressively developed CMS clinical signs associated with dismantlement of NMJs, muscle weakness and fatigability. Of particular interest, forced activation of Wnt/ß-catenin signaling via pharmacological injection of lithium chloride (GSK3 inhibitor) in MuSKΔCRD mice, almost fully rescued both pre- and postsynaptic defects. Taken together, these data revealed that inhibitors of glycogen synthase kinase 3 would be suitable for the treatment of neuromuscular junction-related diseases.

Accordingly the present invention relates to a method of treating a neuromuscular junction-related disease in a subject in need thereof comprising administering the subject with a therapeutically effective amount of at least one inhibitor of glycogen synthase kinase 3 (GSK3).

Treatment may be for any purpose, including the therapeutic treatment of subjects suffering from a neuromuscular junction-related disease, as well as the prophylactic treatment of subjects who do not suffer from a neuromuscular junction-related disease (e.g., subjects identified as being at high risk a neuromuscular junction-related disease). As used herein, the terms "treatment," "treat," and "treating" refer to reversing, alleviating, inhibiting the progress of a disease or disorder as described herein (i.e. a neuromuscular junction-related disease), or delaying, eliminating or reducing the incidence or onset of a disorder or disease as described herein, as compared to that which would occur in the absence of the measure taken. The terms "prophylaxis" or "prophylactic use" and "prophylactic treatment" as used herein, refer to any medical or public health procedure whose purpose is to prevent the disease herein disclosed (i.e. a neuromuscular junction-related disease). As used herein, the terms "prevent", "prevention" and "preventing" refer to the reduction in the risk of acquiring or developing a given condition (i.e. a neuromuscular junction-related disease), or the reduction or inhibition of the recurrence or said condition (i.e. a neuromuscular junction-related disease) in a subject who is not ill, but who has been or may be near a subject with the condition (i.e. a neuromuscular junction-related disease).

The method of the present invention is particularly suitable in the treatment of a wide range of neuromuscular junction-related diseases. As used herein "neuromuscular junction-related disease" refers to a disease resulting from injury at and/or to the neuromuscular junction. A neuromuscular junction-related disease or condition may be, for example, myasthenia gravis, experimentally acquired myasthenia gravis, Lambert-Eaton syndrome, Miller Fischer syndrome, congenital myasthenic syndromes, botulism, organophosphate poisoning, and other toxins that compromise the neuromuscular junction, but also multiple sclerosis, Pompe disease, and Barth syndrome.

As used herein the term "GSK3" has its general meaning in the art and refers to glycogen synthase kinase 3. GSK3 is a protein- serine/threonine kinase whose activity is inhibited by Akt phosphorylation. GSK3 phosphorylates a broad range of substrates including glycogen synthase, several transcription factors, and translation initiation factor. GSK3 is involved in multiple cellular processes including metabolism, cell survival, proliferation, and differentiation. As used herein the term "inhibitor of GSK3" refers to any compound that is able to inhibit the activity or expression of GSK3. The term encompasses any GSK3 inhibitor that is currently known, and/or any GSK3 inhibitor that can be subsequently discovered or created, can be employed with the presently disclosed subject matter.

Several GSK3 inhibitors have been identified and are well known in the art:
- Arfeen M, Bharatam PV. Design of glycogen synthase kinase-3 inhibitors: an overview on recent advancements. Curr Pharm Des. 2013;19(26):4755-75. Review.
- Osolodkin DI, Palyulin VA, Zefirov NS. Glycogen synthase kinase 3 as an anticancer drug target: novel experimental findings and trends in the design of inhibitors. Curr Pharm Des. 2013;19(4):665-79. Review.
- Garcia I, Fall Y, Gómez G. QSAR, docking, and CoMFA studies of GSK3 inhibitors. Curr Pharm Des. 2010;16(24):2666-75. Review.
- Eldar-Finkelman H, Licht-Murava A, Pietrokovski S, Eisenstein M. Substrate competitive GSK-3 inhibitors - strategy and implications. Biochim Biophys Acta. 2010 Mar;1804(3):598-603.
- Takahashi-Yanaga F, Sasaguri T. Drug development targeting the glycogen synthase kinase-3beta (GSK-3beta)-mediated signal transduction pathway: inhibitors of the Wnt/beta-catenin signaling pathway as novel anticancer drugs. J Pharmacol Sci. 2009 Feb;109(2):179-83.
- Duchowicz PR, Castro EA. QSAR studies for the pharmacological inhibition of glycogen synthase kinase-3. Med Chem. 2007 Jul;3(4):393-417. Review.

Example of inhibitors of GSK3 include lithium, in particular lithium chloride, AR-A014418, 4-Acylamino-6-arylfuro[2,3-d]pyrimidines, lithium, SB-415286, P24, CT98014, CHIR98023, ARA014418, AT7519, DM204, Evocapil, LY2090314, Neu120, NP01139, NP03, NP060103, NP07, NP103, SAR502250, VX608 and Zentylor.

Other examples of inhibitors of GSK3 include those described in EP2433636, WO2007031878, WO2007016539, WO2009007457 and WO2005051392.US7595319, US20090041863, US20090233993, EP1739087A1, WO2001070729, WO 03/004472, WO 03/055492, WO 03/082853, WO 06/001754, WO 07/040436, WO 07/040438, WO 07/040439, WO07/040440, WO08/002244 and WO08/992245, WO 00/21927, EP 470490, WO 93/18766, WO 93/18765, EP 397060, WO 98/11103, WO 98/11102, WO 98/04552, WO 98/04551, DE 4243321, DE 4005970, DE 3914764, WO 96/04906, WO 95/07910, DE 4217964, U.S. Pat. No. 5,856,517, U.S. Pat. No. 5,891,901, WO 99/42100, EP 328026, EP 384349, EP 540956, DE 4005969, and EP 508792 which are hereby incorporated by reference.

Particular inhibitors of GSK3 are compounds selected from the group consisting of 3-[7-(2-morpholin-4-ylethoxy)quinazolin-4-yl]-2-oxo-1,3-dihydroindole-5-carbonitrile; 3-[7-(2-methoxyethoxy)quinazolin-4-yl]-2-oxo-1,3-dihydroindole-5-carbonitrile; 3-[7-[2-(2-methoxyethoxy)ethoxy]quinazolin-4-yl]-2-oxo-1,3-dihydroindole-5-carbonitrile; 3-[7-(3-morpholin-4-ylpropoxy)quinazolin-4-yl]-2-oxo-1,3-dihydroindole-5-carbonitrile; 2-hydroxy-3-[5-(4-methylpiperazine-1-carbonyl)pyridin-2-yl]-1H-indole-5-carbonitrile; 1-[(4-methoxyphenyl)methyl]-3-(5-nitro 1,3-thiazol-2-yl)urea; 2-hydroxy-3-[5-[(4-phenylpiperazin-1-yl)methyl]pyridin-2-yl]-1H-indole-5-carbonitrile; 2-hydroxy-3-[5-(morpholin-4-ylmethyl)pyridin-2-yl]-1H-indole-5-carbonitrile; 2-hydroxy-3-[5-(4-methylpiperazin-1-yl)sulfonylpyridin-2-yl]-1H-indole-5-carbonitrile; 2-hydroxy-3-[5-[(4-methylpiperazin-1-yl)methyl]pyridin-2-yl]-1H-indole-5-carbonitrile; 3-[5-(morpholin-4-ylmethyl)pyridin-2-yl]-5-nitro-1H-indol-2-ol; 2-hydroxy-3-[5-(pyrrolidin-1-ylmethyl)pyridin-2-yl]-1H-indole-5-carbonitrile; [6-(2-hydroxy-5-nitro-1H-indol-3-yl)pyridin-3-yl]-(4-methylpiperazin-1-yl)methanone; 2-hydroxy-3-[5-(1-piperidylmethyl)pyridin-2-yl]-1H-indole-5-carbonitrile; 2-hydroxy-3-[5-(morpholin-4-ylmethyl)pyridin-2-yl]-1H-indole-6-carbonitrile; 2-hydroxy-3-[5-(4-methylpiperazin-1-yl)sulfonylpyridin-2-yl]-1H-indole-6-carbonitrile; 3-fluoro-3-[5-(morpholin-4-ylmethyl)pyridin-2-yl]-2-oxo-1H-indole-6-carbonitrile; [4-[5-(4-methoxyphenyl)-2,7,9-triazabicyclo[4.3.0]nona-1,3,5,7-tetraen-8-yl]phenyl]-(4-methylpiperazin-1-yl)methanone; 3-(4-methoxyphenyl)-N-(2-morpholin-4-ylethyl)-5,7-diazabicyclo[4.3.0]nona-1,3,5,8- tetraene-9-carboxamide; 3-(4-chlorophenyl)-N-(2-morpholin-4-ylethyl)-5,7-diazabicyclo[4.3.0]nona-1,3,5,8- tetraene-9-carboxamide; 5-fluoro-N-[4-(4-methylpiperazin-1-yl)sulfonylphenyl]-4-(2-methyl-3-propan-2-yl-imidazol-4-yl)pyrimidin-2-amine; [4-[[5-fluoro-4-(2-methyl-3-propan-2-yl-imidazol-4-yl)pyrimidin-2-yl]amino]phenyl]- (4-methylpiperazin-1-yl)methanone; [4-[[5-fluoro-4-(2-methyl-3-propan-2-yl-imidazol-4-yl)pyrimidin-2-yl]amino]phenyl]-phenyl-methanone; N-(3-methoxypropyl)-8-[4-(trifluoromethyl)phenyl]-2,7,9-triazabicyclo[4.3.0]nona-1,3,5,7-tetraene-5-carboxamide; 5-(4-methoxyphenyl)-8-[4-(1-piperidylmethyl)phenyl]-2,7,9-triazabicyclo[4.3.0]nona-1,3,5,7-tetraene; N-(3-methoxypropyl)-8-[4-(morpholin-4-ylmethyl)phenyl]-2,7,9-triazabicyclo[4.3.0]nona-1,3,5,7-tetraene-5-carboxamide; [4-[[5-fluoro-4-(2-methyl-3-propan-2-yl-imidazol-4-yl)pyrimidin-2-yl]amino]phenyl]-pyridin-2-yl-methanone; [4-[[4-(3-cyclohexyl-2-methyl-imidazol-4-yl)-5-fluoro-pyrimidin-2-yl]amino]phenyl]-(4-i o methylpiperazin-1-yl)methanone; [4-[[5-fluoro-4-[2-methyl-3-(oxan-4-yl)imidazol-4-yl]pyrimidin-2-yl]amino]phenyl]-(4-methylpiperazin-1-yl)methanone; 5-fluoro-4-[2-methyl-3-(oxan-4-yl)imidazol-4-yl]-N-[4-(4-methylpiperazin-1-yl)sulfonylphenyl]pyrimidin-2-amine; 4-(2,3-dimethylimidazol-4-yl)-5-fluoro-N-[4-(morpholin-4-ylmethyl)phenyl]pyrimidin-2-amine; [4-[5-(4-chlorophenyl)-2,7,9-triazabicyclo[4.3.0]nona-1,3,5,7-tetraen-8-yl]phenyl]-(4-methylpiperazin-1-yl)methanone; N-(3-methoxypropyl)-8-[3-(2,2,3,3-tetrafluoropropoxymethyl)phenyl]-2,7,9-20 triazabicyclo[4.3.0]nona-1,3,5,7-tetraene-5-carboxamide; [4-[[5-fluoro-4-[2-methyl-3-(oxan-4-yl)imidazol-4-yl]pyrimidin-2-yl]amino]phenyl]-pyridin-2-yl-methanone; [4-[[4-(2,3-dimethylimidazol-4-yl)-5-fluoro-pyrimidin-2-yl]amino]phenyl]-pyridin-2-yl-methanone; 5-fluoro-4-[2-methyl-3-(oxan-4-yl)imidazol-4-yl]-N-[4-(morpholin-4-ylmethyl)phenyl]pyrimidin-2-amine; [4-[[4-(2,3-dimethylimidazol-4-yl)-5-fluoro-pyrimidin-2-yl]amino]phenyl]-(4-methylpiperazin-1-yl)methanone; 4-(2,3-dimethylimidazol-4-yl)-5-fluoro-N-[4-[(4-methylpiperazin-1- 30 yl)methyl]phenyl]pyrimidin-2-amine; 5-fluoro-4-[2-methyl-3-(oxan-4-yl)imidazol-4-yl]-N-(4-methylsulfonylphenyl)pyrimidin-2-amine; 5-fluoro-4-[2-methyl-3-(oxan-4-yl)imidazol-4-yl]-N-(6-methylpyridin-3-yl)pyrimidin-2- amine; [4-[[4-(2,3-dimethylimidazol-4-yl)-5-fluoro-pyrimidin-2-yl]amino]-2-(trifluoromethoxy)phenyl]-(4-methylpiperazin-1-yl)methanone; 5-fluoro-N-[4-(4-methylpiperazin-1-yl)sulfonylphenyl]-4-[3-(oxan-4-yl)-2-(trifluoromethyl)imidazol-4-yl]pyrimidin-2-amine; azetidin-1-yl-[4-[[5-fluoro-4-[2-methyl-3-(oxan-4-yl)imidazol-4-yl]pyrimidin-2-yl]amino]phenyl]methanone; 5-fluoro-N-[3-methyl-4-(morpholin-4-ylmethyl)phenyl]-4-[2-methyl-3-(oxan-4-yl)imidazol-4-yl]pyrimidin-2-amine; 5-fluoro-N-[4-(morpholin-4-ylmethyl)phenyl]-4-[3-(oxan-4-yl)-2-(trifluoromethyl)imidazol-4-yl]pyrimidin-2-amine; 5-fluoro-N-[4-(4-methylpiperazin-1-yl)sulfonylphenyl]-4-[3-(oxan-4-yl)imidazol-4-yl]pyrimidin-2-amine; [4-[[5-fluoro-4-[3-(oxan-4-yl)-2-(trifluoromethyl)imidazol-4-yl]pyrimidin-2-yl]amino]phenyl]-(4-methylpiperazin-1- yl)methanone; 5-[5-fluoro-2-[[4-(4-methylpiperazin-1-yl)sulfonylphenyl]amino]pyrimidin-4-yl]-1-(oxan-4-yl)imidazole-2-carbonitrile; 5-fluoro-N-[4-(4-methylpiperazin-1-yl)sulfonylphenyl]-4-[3-methyl-2-(trifluoromethyl)imidazol-4-yl]pyrimidin-2-amine; 5-fluoro-4-[3-methyl-2-(trifluoromethyl)imidazol-4-yl]-N-[4-(morpholin-4-ylmethyl)phenyl]pyrimidin-2-amine; [4-[[5-fluoro-4-[3-methyl-2-(trifluoromethyl)imidazol-4-yl]pyrimidin-2-yl]amino]phenyl]-(4-methylpiperazin-1-yl)methanone; N-(2-cyanoethyl)-3-[5-(4-methoxyphenyl)-2,7,9-triazabicyclo[4.3.0]nona-1,3,5,7-tetraen-8-yl]benzamide; 5-fluoro-4-[2-methyl-3-(oxan-4-yl)imidazol-4-yl]-N-[4-methylsulfonyl-3-(trifluoromethyl)phenyl]pyrimidin-2-amine; azetidin-1-yl-[4-[8-[4-(morpholin-4-ylmethyl)phenyl]-2,7,9-triazabicyclo[4.3.0]nona-1,3,5,7-tetraen-5-yl]phenyl]methanone; 8-[4-(morpholin-4-ylmethyl)phenyl]-N-pyridin-3-yl-2,7,9-triazabicyclo[4.3.0]nona-1 ,3,5,7-tetraene-5-carboxamide; 2-[3-[8-[4-(4-methylpiperazine-1-carbonyl)phenyl]-2,7,9-triazabicyclo[4.3.0]nona-1,3,5,7-tetraen-5-yl]phenoxy]acetonitrile; 5-fluoro-N-(4-methylsulfbnylphenyl)-4-[3-propan-2-yl-2-(trifluoromethyl)imidazol-4-yl]pyrimidin-2-amine; 5-fluoro-N-[4-(4-methylpiperazin-1-yl)sulfonylphenyl]-4-[3-propan-2-yl-2-(trifluoromethyl)imidazol-4-yl]pyrimidin-2-amine; [4-[[5-fluoro-4-[3-propan-2-yl-2-(trifluoromethyl)imidazol-4-yl]pyrimidin-2-yl]amino]phenyl]-(4-methylpiperazin-1-yl)methanone; 4-[2-methyl-3-(oxan-4-yl)imidazol-4-yl]-N-(4-methylsulfonylphenyl)pyrimidin-2-amine; 4-[2-methyl-3-(oxan-4-yl)imidazol-4-yl]-N-[4-(4-methylpiperazin-1-yl)sulfonylphenyl]pyrimidin-2-amine; [4-[[4-[2-methyl-3-(oxan-4-yl)imidazol-4-yl]pyrimidin-2-yl]amino]phenyl]-(4-methylpiperazin-1-yl)methanone; 4-[2-methyl-3-(oxan-4-yl)imidazol-4-yl]-N-[4-(morpholin-4-ylmethyl)phenyl]pyrimidin-2-amine; 5-(4-methoxyphenyl)-8-(3-methylsulfonylphenyl)-2,7,9-triazabicyclo[4.3.0]nona-1,3,5, 7-tetraene; [4-[5-(3-fluoro-4-methoxy-phenyl)-2,7,9-triazabicyclo[4.3.0]nona-1,3,5,7-tetraen-8-yl]phenyl]-(4-methylpiperazin-1-yl)methanone; 5-fluoro-4-[2-methyl-3-(oxan-4-yl)imidazol-4-yl]-N-(6-methylsulfonylpyridin-3-yl)pyrimidin-2-amine; (2,6-dimethylmorpholin-4-yl)-[4-[[5-fluoro-4-[2-methyl-3-(oxan-4-yl)imidazol-4-yl]pyrimidin-2-yl]amino]phenyl]methanone; [5-[[5-fluoro-4-[2-methyl-3-(oxan-4-yl)imidazol-4-yl]pyrimidin-2-yl]amino]pyridin-2-yl]-(4-methylpiperazin-1-yl)methanone; 5-fluoro-4-[2-methyl-3-(oxan-4-yl)imidazol-4-yl]-N-[4-(1-morpholin-4- ylethyl)phenyl]pyrimidin-2-amine; 5-fluoro-N-(4-methylsulfbnylphenyl)-4-[3-(oxan-4-yl)-2-(trifluoromethyl)imidazol-4-yl]pyrimidin-2-amine; azetidin-1-yl-[2-chloro-4-[[4-(2,3-dimethylimidazol-4-yl)-5-fluoro-pyrimidin-2-yl]amino]phenyl]methanone; azetidin-1-yl-[4-[[4-(2,3-dimethylimidazol-4-yl)-5-fluoro-pyrimidin-2-yl]amino]-2-methyl-phenyl]methanone; azetidin-1-yl-[5-[[4-(2,3-dimethylimidazol-4-yl)-5-fluoro-pyrimidin-2-yl]amino]pyridin-2-yljmethanone; azetidin-1-yl-[4-[[4-(2,3-dimethylimidazol-4-yl)-5-fluoro-pyrimidin-2-yl]amino]-2-(trifluoromethoxy)phenyl]methanone; azetidin-1-yl-[3-chloro-5-[[4-(2,3- dimethylimidazol-4-yl)-5-fluoro-pyrimidin-2-yl]amino]pyridin-2-yljmethanone; 5-fluoro-4-[2-methyl-3-(oxan-4-yl)imidazol-4-yl]-N-[6-(morpholin-4-ylmethyl)pyridin-3-yl]pyrimidin-2-amine; 5-fluoro-4-[2-methyl-3-(oxan-4-yl)imidazol-4-yl]-N-(6-propan-2-ylsulfonylpyridin-3- yl)pyrimidin-2-amine; azetidin-1-yl-[3-chloro-5-[[5-fluoro-4-[2-methyl-3-(oxan-4-yl)imidazol-4-yl]pyrimidin-2-yl]amino]pyridin-2-yljmethanone; N-(6-ethylsulfonylpyridin-3-yl)-5-fluoro-4-[2-methyl-3-(oxan-4-yl)imidazol-4-yl]pyrimidin-2-amine; [3-chloro-5-[[5-fluoro-4-[3-(oxan-4-yl)-2-(trifluoromethyl)imidazol-4-yl]pyrimidin-2-yl]amino]pyridin-2-yl]-(4-methylpiperazin-1-yl)methanone; 5-[[5-fluoro-4-[2-methyl-3-(oxan-4-yl)imidazol-4-yl]pyrimidin-2-yl]amino]-N-methyl-N-propan-2-yl-pyridine-2-carboxamide; N-ethyl-5-[[5-fluoro-4-[2-methyl-3-(oxan-4-yl)imidazol-4-yl]pyrimidin-2-yl]amino]-N-methyl-pyridine-2-carboxamide; 3-[4-[8-[4-(morpholin-4-ylmethyl)phenyl]2,7,9-triazabicyclo[4.3.0]nona-1,3,5,7-tetraene-5-carbonyl]piperazin-1-yl]propanenitrile; [3-chloro-5-[[5-fluoro-4-[3-methyl-2-(trifluoromethyl)imidazol-4-yl]pyrimidin-2-yl]amino]pyridin-2-yl]-(4-methylpiperazin-1-yl)methanone; [3-chloro-5-[[5-fluoro-4-[3-methyl-2-(trifluoromethyl)imidazol-4-yl]pyrimidin-2-yl]amino]pyridin-2-yl]-(1-piperidyl)methanone; azetidin-1-yl-[3-chloro-5-[[5-fluoro-4-[3-methyl-2-(trifluoromethyl)imidazol-4-yl]pyrimidin-2-yl]amino]pyridin-2-yl]methanone; 5-fluoro-4-[2-methyl-3-(oxan-4-yl)imidazol-4-yl]-N-[6-(4-methylpiperazin-1-yl)sulfonylpyridin-3-yl]pyrimidin-2-amine; N-[6-[(4,4-difluoro-1-piperidyl)methyl]pyridin-3-yl]-5-fluoro-4-[2-methyl-3-(oxan-4-yl)imidazol-4-yl]pyrimidin-2-amine; 5-fluoro-4-[2-methyl-3-(oxan-4-yl)imidazol-4-yl]-N-(oxan-4-yl)pyrimidin-2-amine; 1-[4-[[5-fluoro-4-[2-methyl-3-(oxan-4-yl)imidazol-4-yl]pyrimidin-2-yl]amino]-1-piperidyl]ethanone; [4-[[5-fluoro-4-[2-methyl-3-(oxan-4-yl)imidazol-4-yl]pyrimidin-2-yl]amino]-1-piperidyl]- phenyl-methanone; 1-[4-[[5-fluoro-4-[2-methyl-3-(oxan-4-yl)imidazol-4-yl]pyrimidin-2-yl]amino]-1-piperidyl]-2-phenyl-ethanone; benzyl 4-[[5-fluoro-4-[2-methyl-3-(oxan-4-yl)imidazol-4-yl]pyrimidin-2-yl]amino]piperidine-1-carboxylate; 5-fluoro-4-[2-methyl-3-(oxan-4-yl)imidazol-4-yl]-N-(1-methylsulfonyl-4-piperidyl)pyrimidin-2-amine; N-[1-(benzenesulfonyl)-4-piperidyl]-5-fluoro-4-[2-methyl-3-(oxan-4-yl)imidazol-4- yl]pyrimidin-2-amine; 5-[[5-fluoro-4-[2-methyl-3-(oxan-4-yl)imidazol-4-yl]pyrimidin-2-yl]amino]-N,N-dimethyl-pyridine-2-sulfonamide; an isomer, a metabolite , a prodrug or a pharmaceutically acceptable salt thereof, or a solvate or a solvate of a pharmaceutically acceptable salt thereof.

An "inhibitor of expression" refers to a natural or synthetic compound that has a biological effect to inhibit the expression of a gene.

In some embodiments, said inhibitor of gene expression is a siRNA, an antisense oligonucleotide or a ribozyme.

Inhibitors of gene expression for use in the present invention may be based on antisense oligonucleotide constructs. Anti-sense oligonucleotides, including anti-sense RNA molecules and anti-sense DNA molecules, would act to directly block the translation of the targeted mRNA by binding thereto and thus preventing protein translation or increasing mRNA degradation, thus decreasing the level of the targeted protein (i.e. GSK3), and thus activity, in a cell. For example, antisense oligonucleotides of at least about 15 bases and complementary to unique regions of the mRNA transcript sequence encoding the target protein can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of gene expression for use in the present invention. Gene expression can be reduced by contacting the tumor, subject or cell with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known (e.g. see Tuschi, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of the targeted mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hematopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Typically the inhibitor of GSK3 is administered to the patient in a therapeutically effective amount.

By a "therapeutically effective amount" of the inhibitor of GSK3 as above described is meant a sufficient amount of the compound. It will be understood, however, that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typicially, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the invention, the inhibitor of GSK3 is administered to the subject in the form of a pharmaceutical composition. Typically, the inhibitor of GSK3 may be combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The inhibitor of GSK3 can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

The invention will be further illustrated by the following figures and examples. However, these examples and figures should not be interpreted in any way as limiting the scope of the present invention.

### FIGURES:

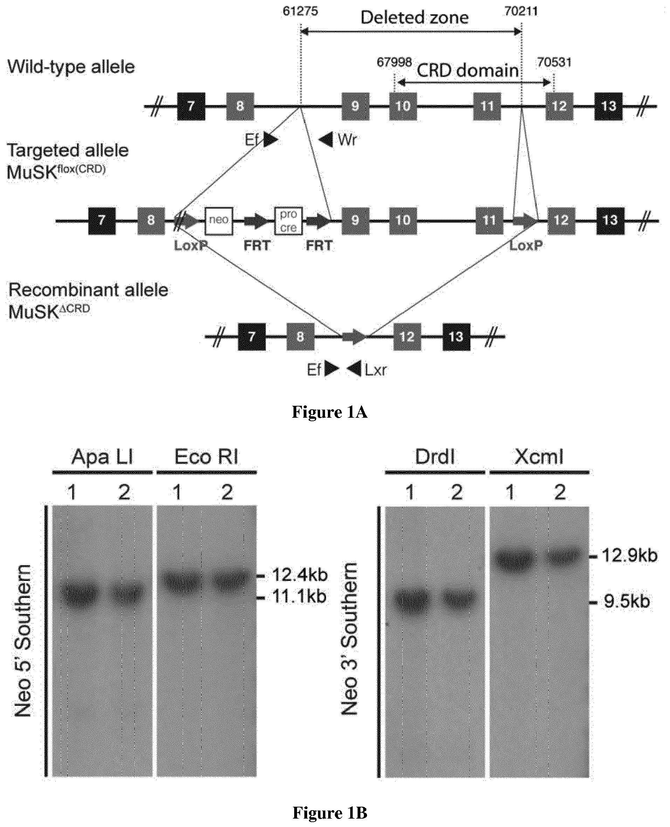

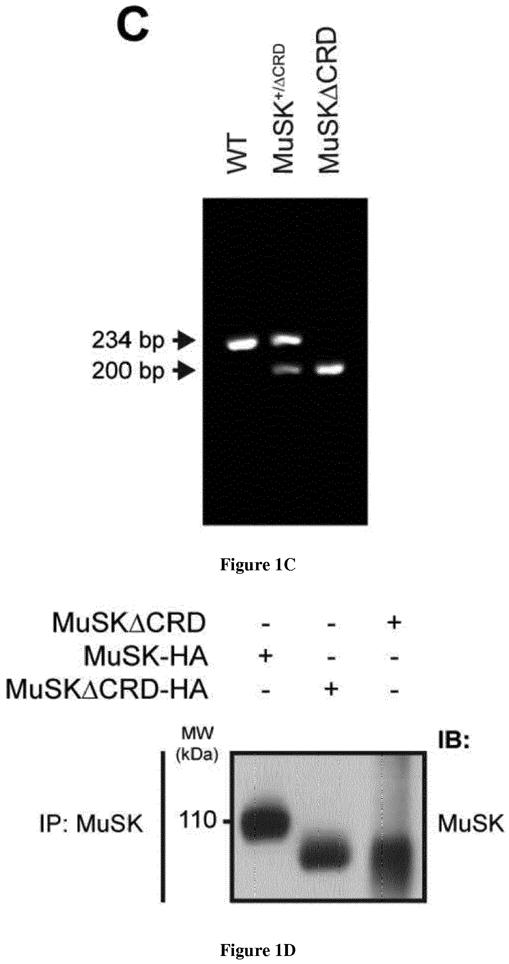

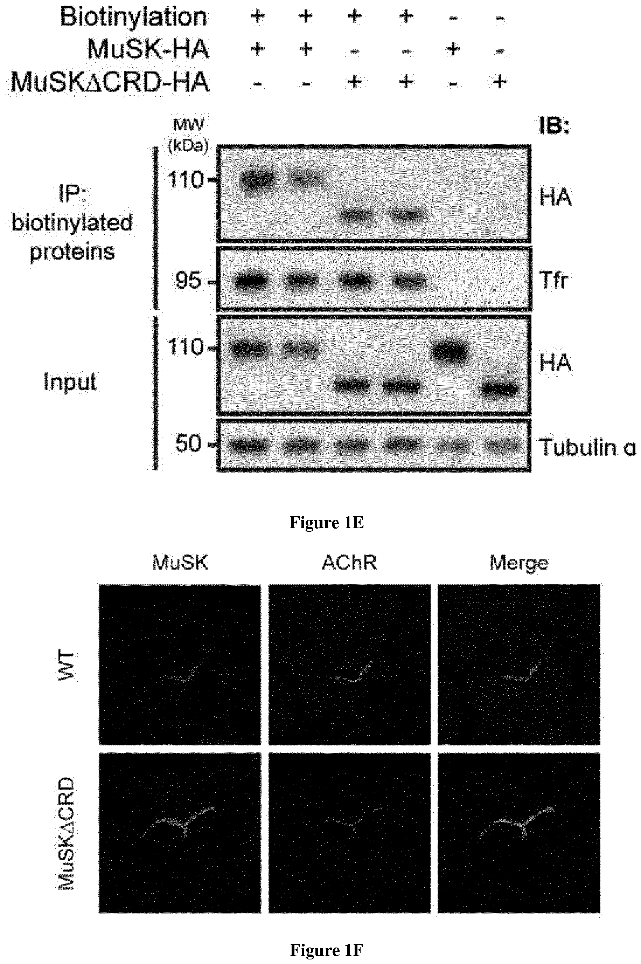

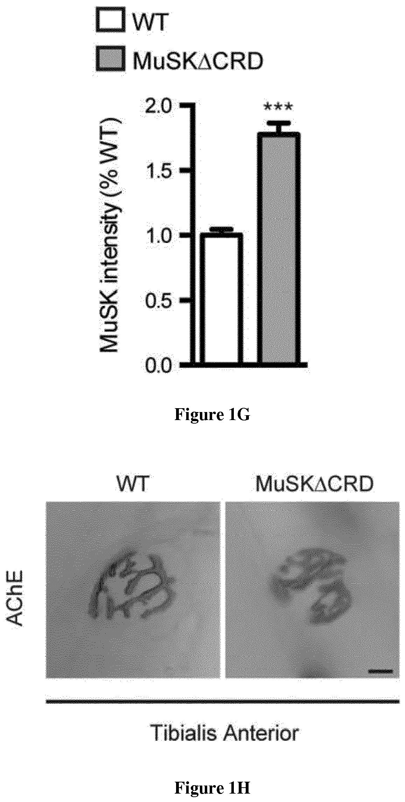

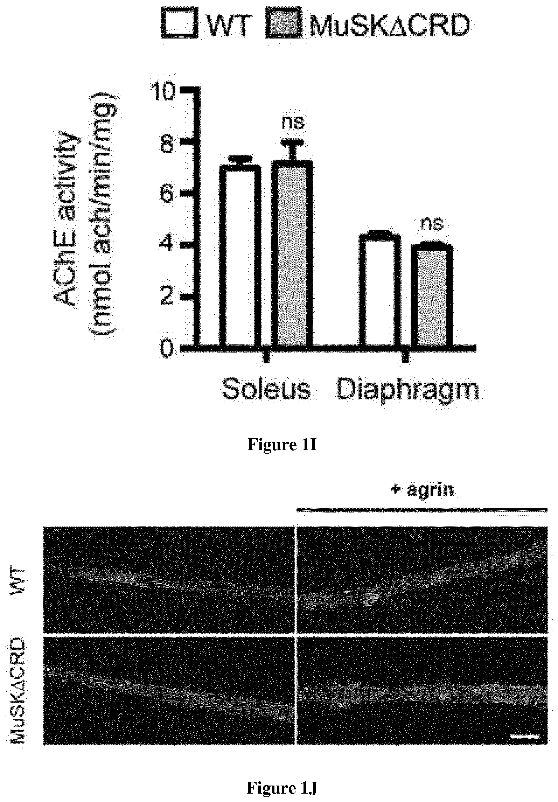

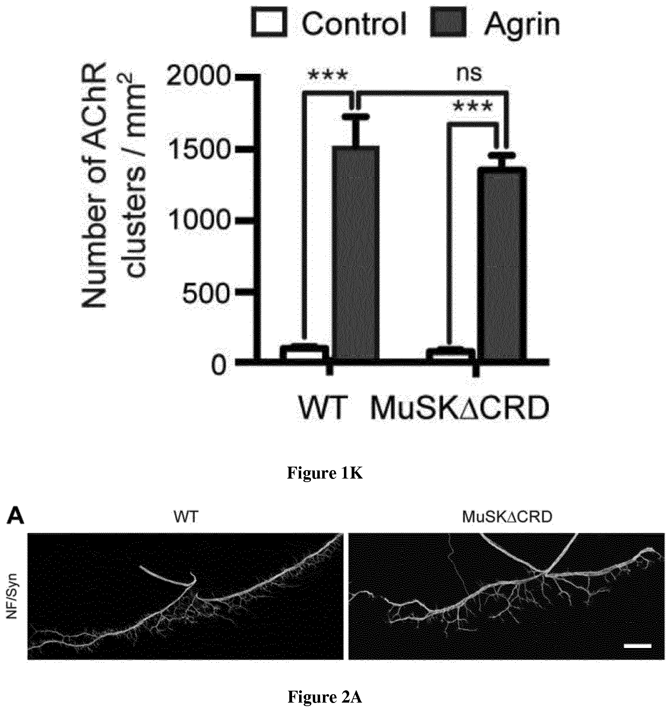

**Figure 1****. Generation of MuSKΔCRD transgenic mice.**
   (A) Schematic representation of the wild type (WT), MuSK^{flox}(CRD), and recombined MuSKΔCRD alleles. Arrowheads, primers for genotyping. (B) Examples of Neo Southern blot hybridization analysis of the injected ES mutant clones. (C) Genotyping by PCR. The 234 and 200 bp bands represent WT and MuSKΔCRD alleles, respectively. (D) Western blot of MuSK or MuSKΔCRD using MuSK antibodies in HEK293T cells transfected with MuSK-HA or MuSKΔCRD-HA or in MuSKΔCRD primary myotubes after MuSK immunoprecipitation. (E) Western blot of cell surface and total MuSK-HA and MuSKΔCRD-HA in HEK293T cells transfected with MuSK-HA or MuSKΔCRD-HA. Transferrin receptor (Tfr) and α -tubulin were used as a loading control for biotinylated proteins and input respectively. Transfection of MuSK-WT-HA or MuSKΔCRD-HA was performed in duplicate. (F) Confocal images of P60 WT or MuSKΔCRD TA muscle cross sections stained with MuSK antibody (red) together with α-BTX (AChR, green). (G) Quantification of WT and mutated MuSK signal intensities at the synapse. (H) Koelle's histochemical staining of acetylcholinesterase (AChE) performed on isolated TA muscle fibers from P90 WT and MuSKΔCRD. (I) Quantification of AChE activity extracted from P90 WT and MuSKΔCRD soleus and diaphragm. (J) Examples of myotubes isolated from WT or MuSKΔCRD primary cultures, treated or not with recombinant agrin and stained with α-BTX. (K) Quantitative analysis of the number of AChR clusters in WT and MuSKΔCRD myotubes. Data are shown as mean ± SEM. ***p < 0.001. ns, non significant. N = 3 independent experiments. Scale bar in the merged image in E, 20 µm; in G, 50 µm.

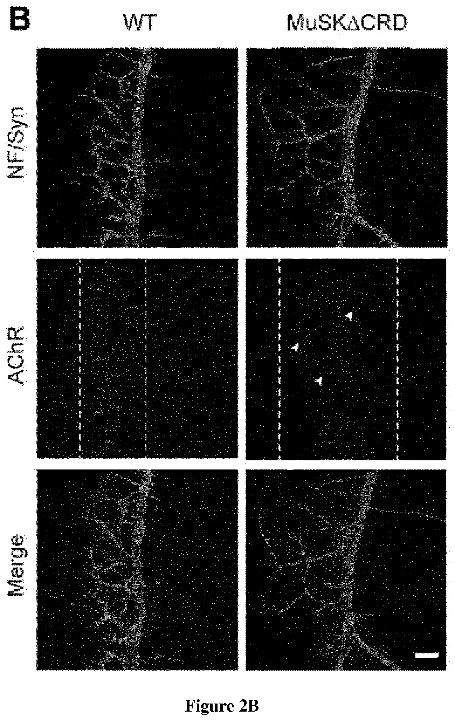

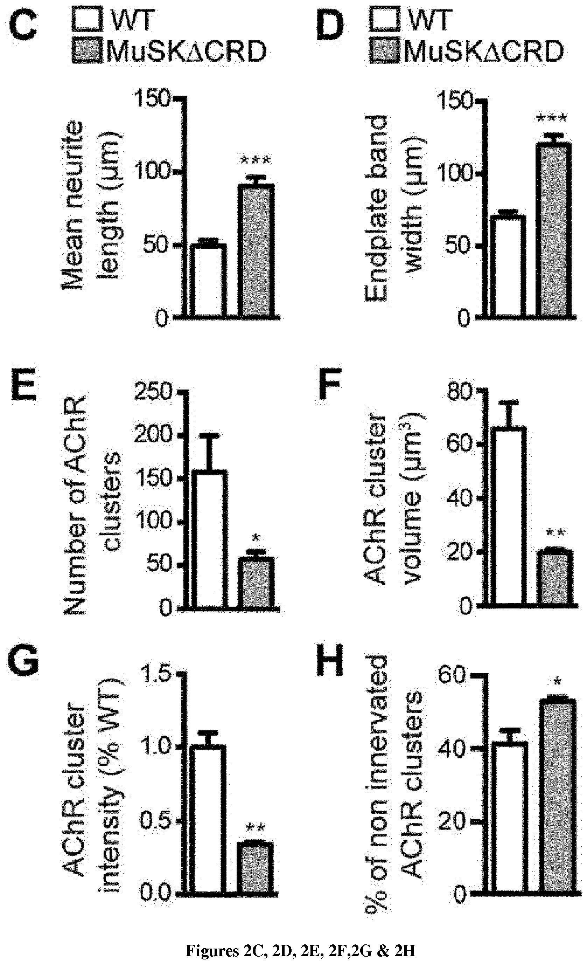

**Figure 2****. Impaired muscle prepatterning in MuSKΔCRD embryos.**
   (A-B) Confocal images of whole mount left hemidiaphragms from E14 WT and MuSKΔCRD embryos stained with neurofilament (NF, red) and synaptophysin (Syn, red) antibodies (A-B) together with α-BTX (AChRs, green, B). Arrowheads, AChR clusters. White dashed lines delineate the synaptic endplate band and include most AChR clusters. (C-H) Quantitative analysis of the mean neurite length (C), the endplate band width (D), the AChR clusters number (E), volume (F), intensity (G) and non innervated AChR clusters (H). Number of AChR clusters analyzed: 790 in WT and 461 in MuSKΔCRD. Data are shown as mean ± SEM. *p<0.05, **p<0.01, and ***p<0.001, N = 4 embryos per genotype, Mann-Whitney U test. Scale bar in the merged image in A, 300 µm; in B, 50 µm.

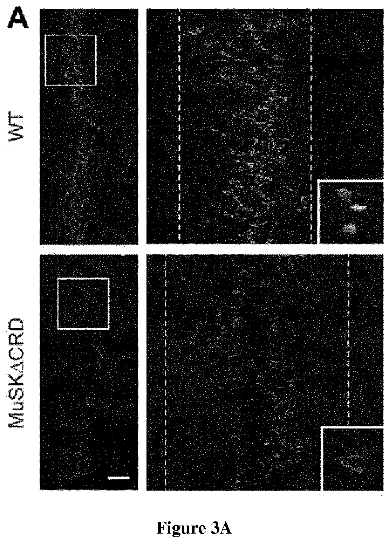

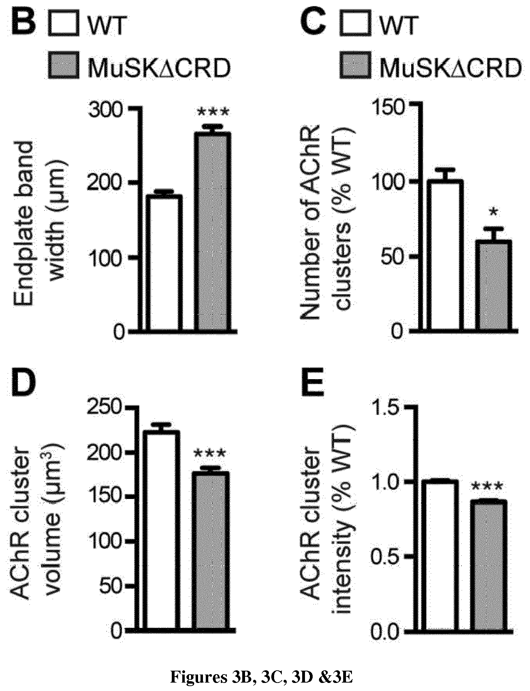

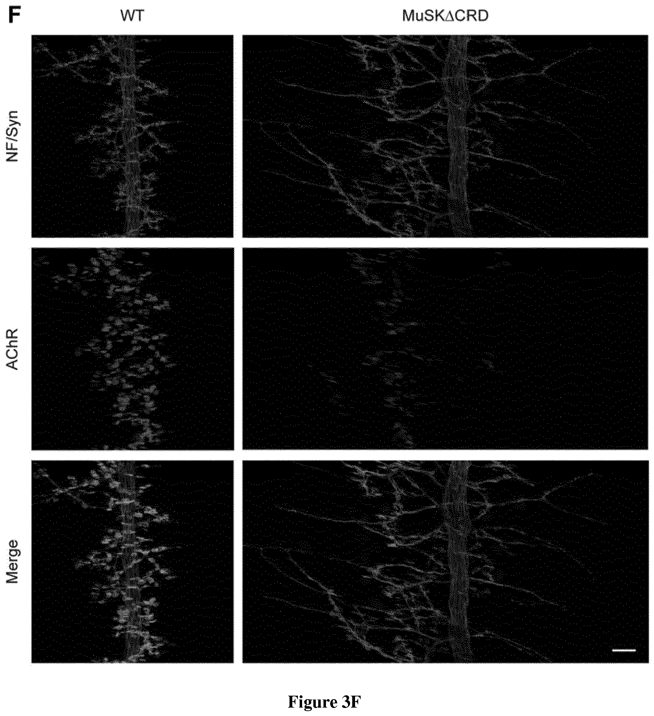

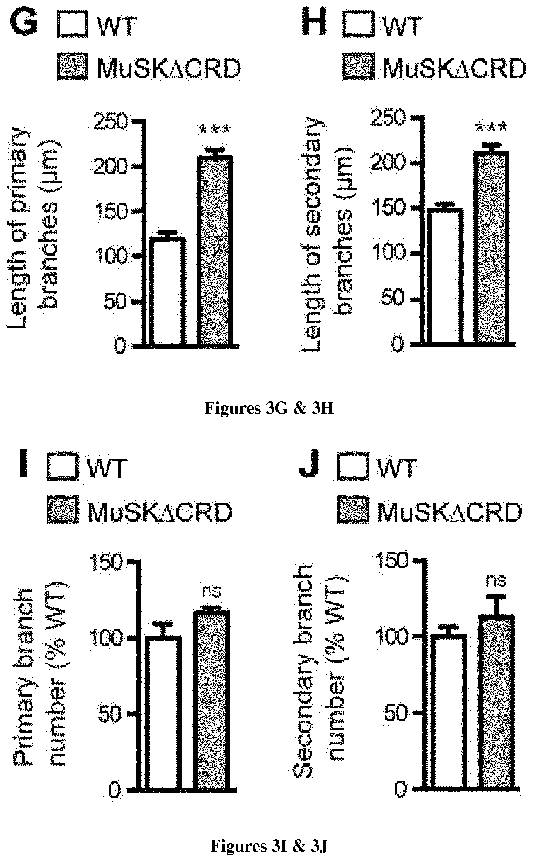

**Figure 3****. Aberrant NMJ formation in E18.5 MuSKΔCRD embryos.**
   (A) Confocal images of whole mount left hemidiaphragms from E18.5 WT and MuSKΔCRD embryos stained with α-BTX to visualize AChR clusters. Right panels show enlarged images of boxed regions in left panel. White dashed lines delineate the synaptic endplate band and include most AChR clusters. Insets in right panels are higher magnification views of AChR clusters. (B-E) Quantifications of the endplate band width (B), the AChR clusters number (C), volume (D) and intensity (E). Numbers of AChR clusters analyzed: 806 in WT and 604 in MuSKΔCRD. (F) Confocal images of whole mount left hemidiaphragms from E18.5 WT and MuSKΔCRD embryos stained as in Figure 2B. (G-J) Quantitative analysis of the length (G-H) and the number (I-J) of primary and secondary nerve branches. Number of primary branches analyzed: 273 in WT and 309 in MuSKΔCRD, secondary branches: 266 in WT and 310 in MuSKΔCRD. Data are shown as mean ± SEM. *p<0.05, **p<0.01, and ***p<0.001, ns: non significant. N = 6 embryos per genotype, Mann-Whitney U test. Scale bar in the merged image in A, 300 µm; in F, 50 µm.

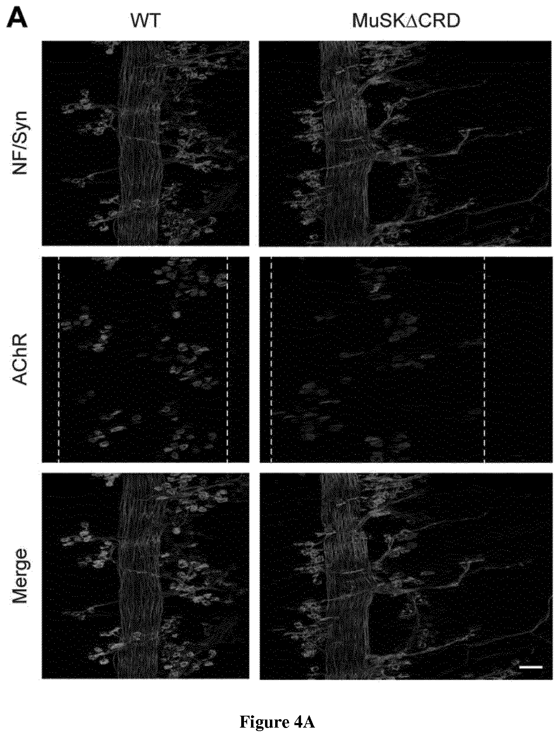

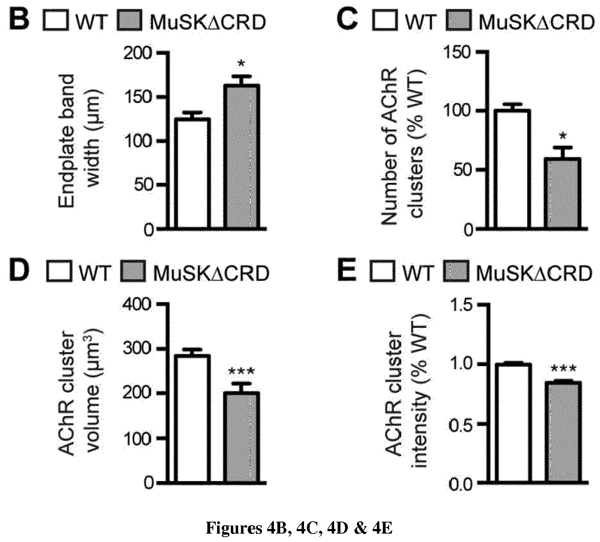

**Figure 4****. Diaphragm innervation defects in P5 MuSKΔCRD mice.**
   (A) Confocal images of whole mount P5 WT and MuSKΔCRD left hemidiaphragms stained as Figure 2B. White dashed lines delineate the synaptic endplate band and include most AChR clusters. (B-E) Quantitative analysis of the endplate band width (B), the AChR clusters number (C), volume (D) and intensity (E). Numbers of AChR clusters tested: 263 in WT and 120 in MuSKΔCRD. Data are shown as mean ± SEM. *p<0.05 and ***p<0.001. N = 4 embryos per genotype, Mann-Whitney U test. Scale bar, 50 µm.

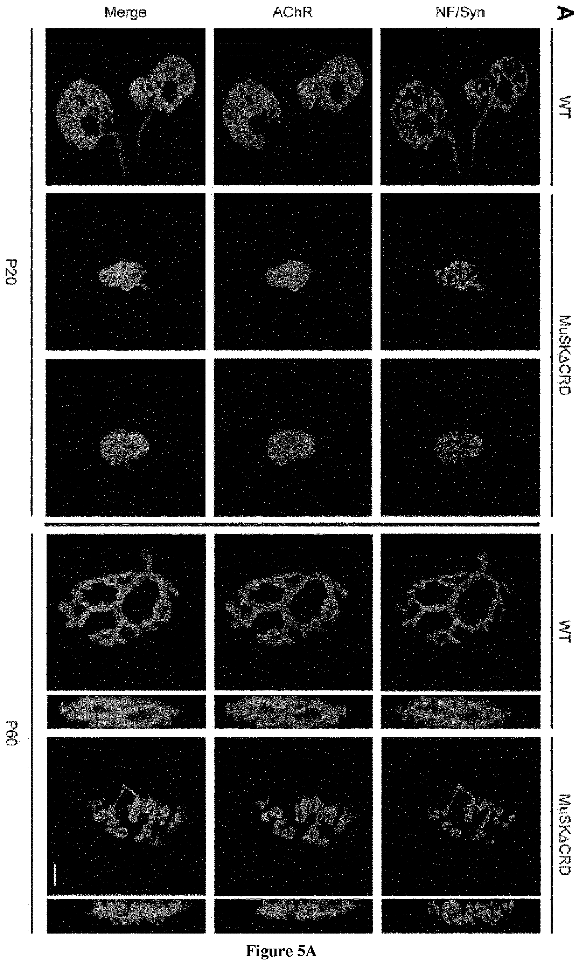

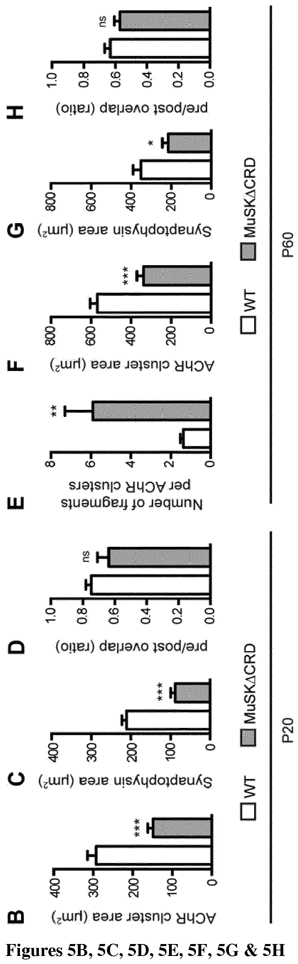

**Figure 5****. Immature and fragmented NMJs in MuSKΔCRD adult mice.**
   Whole mount isolated muscle fibers from P20 and P60 WT and MuSKΔCRD TA were stained with neurofilament (NF, red) and synaptophysin (Syn, red) antibodies together with α-BTX (AChRs, green). (A) Confocal images of synapses from P20 and P60 WT and MuSKΔCRD mice. For P60 NMJs, top views of the reconstructed image are represented on the right side. (B-D) Quantification analysis of the AChR cluster area (B), the synaptophysin area (C) and overlap area of pre and postsynaptic elements (D) in P20 WT and MuSKΔCRD mice. (E-H) Quantification analyses of the number of fragments per AChR clusters (E), the AChR cluster area (F), the synaptophysin area (G) and the overlap ratio of pre- and postsynaptic elements (H) in P60 WT and MuSKΔCRD mice. Data are shown as mean ± SEM of at least 50 NMJs. *p<0.05, ***p<0.001, ns: non significant. N = 6 animals per genotype, Mann-Whitney U test. Scale bar in the merged image, 10 µm.

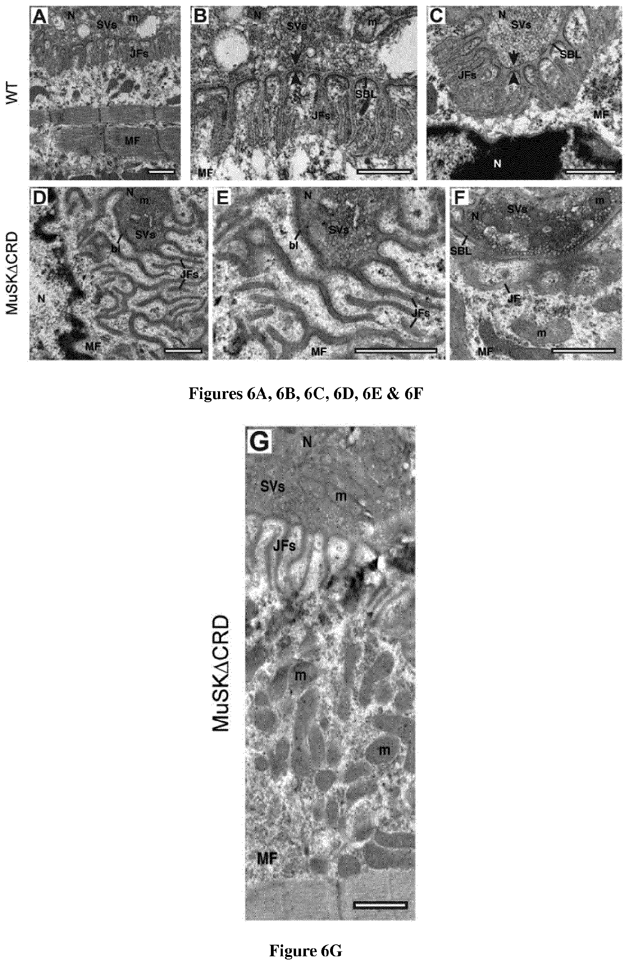

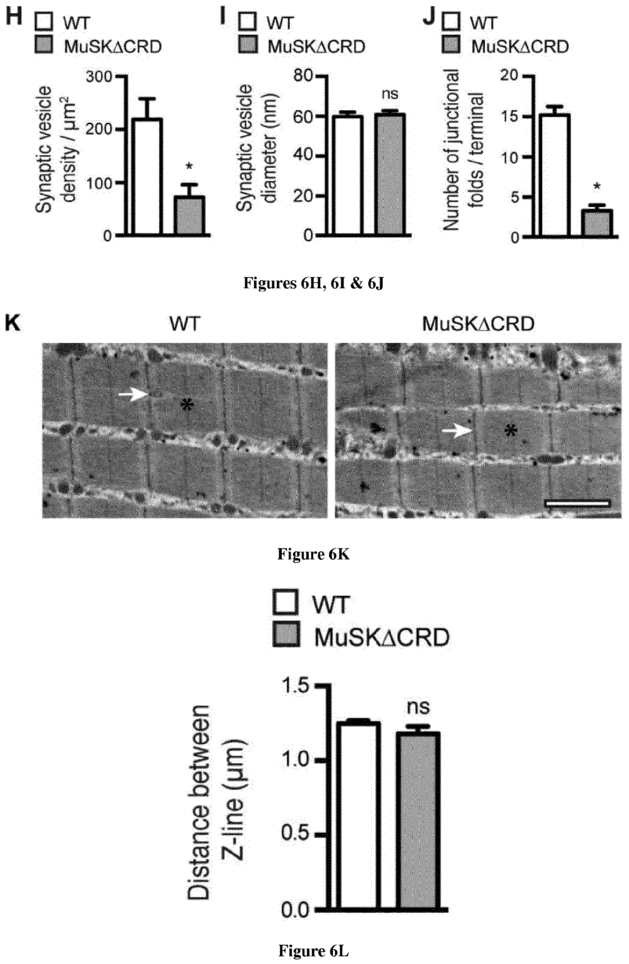

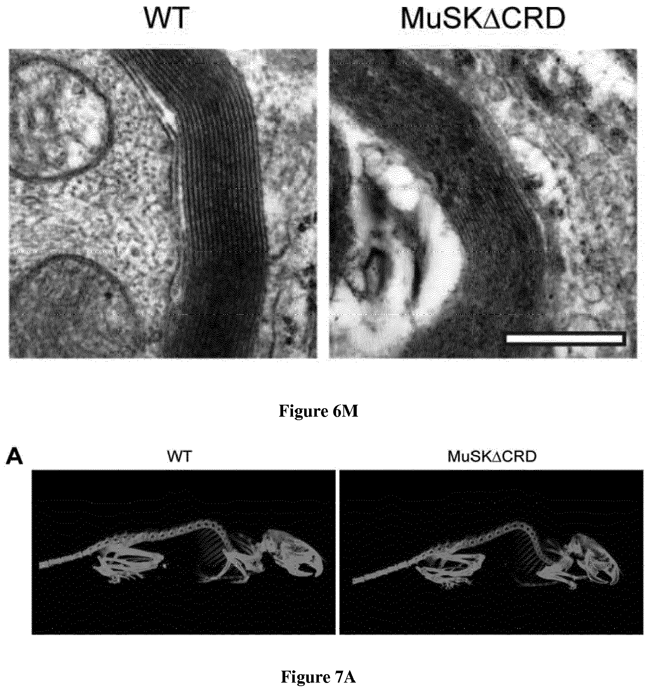

**Figure 6****. Disorganised NMJ ultrastructures in MuSKΔCRD mice.**
   (A-G) Representative electron micrographs (EM) of P120 WT and MuSKΔCRD TA NMJs. A-C, examples of WT NMJs. D-G, examples of MuSKΔCRD NMJs. B and E are higher magnification views of A and D respectively. (H-J) Quantification analyses of the synaptic vesicle density (H), diameter (I) and the number of JFs (J) in MuSKΔCRD compared to WT mice. (K) Representative EM of P120 WT and MuSKΔCRD TA structure. (L) Quantification of the distance between Z-lines in MuSKΔCRD and WT mice. (M) Representative EM of myelin sheath in P120 WT and MuSKΔCRD TA. Data are shown as mean ± SEM. ns: non significant. N = 4 animals per genotype, Mann-Whitney U test. N, nerve; MF, muscle fiber; SVs, synaptic vesicles; JFs, junctional folds; m, mitochondria; SBL, synaptic basal lamina; arrow, presynaptic membrane; arrowhead, postsynaptic membrane; white arrow, Z-line; star, M-line. Scale bar in A-G, 500 nm; in H and J, 1 µm.

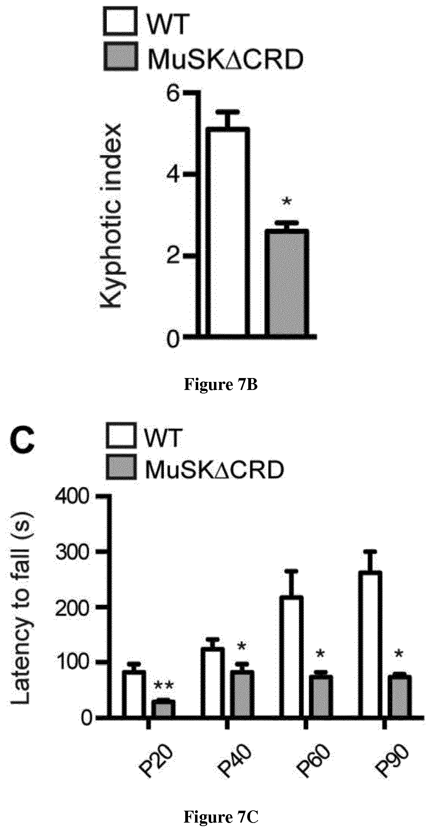

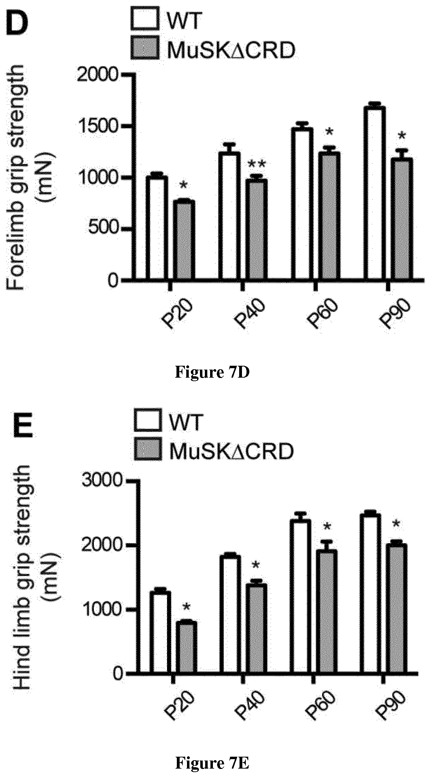

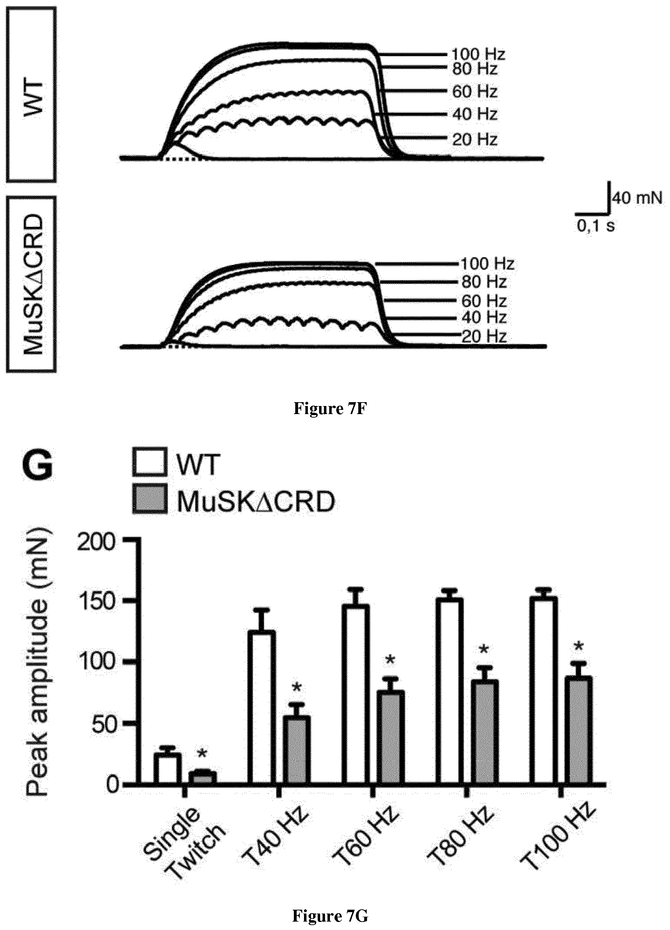

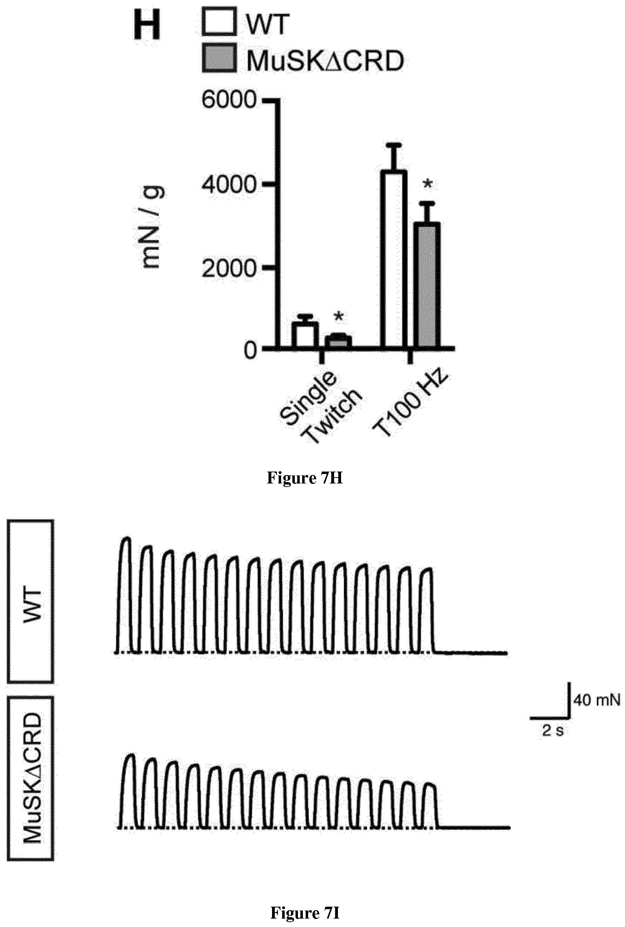

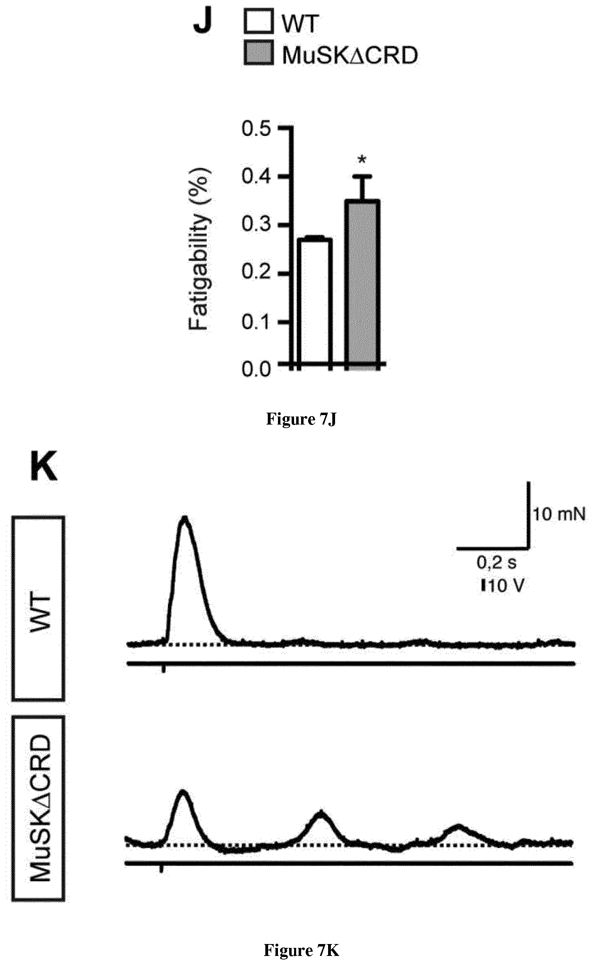

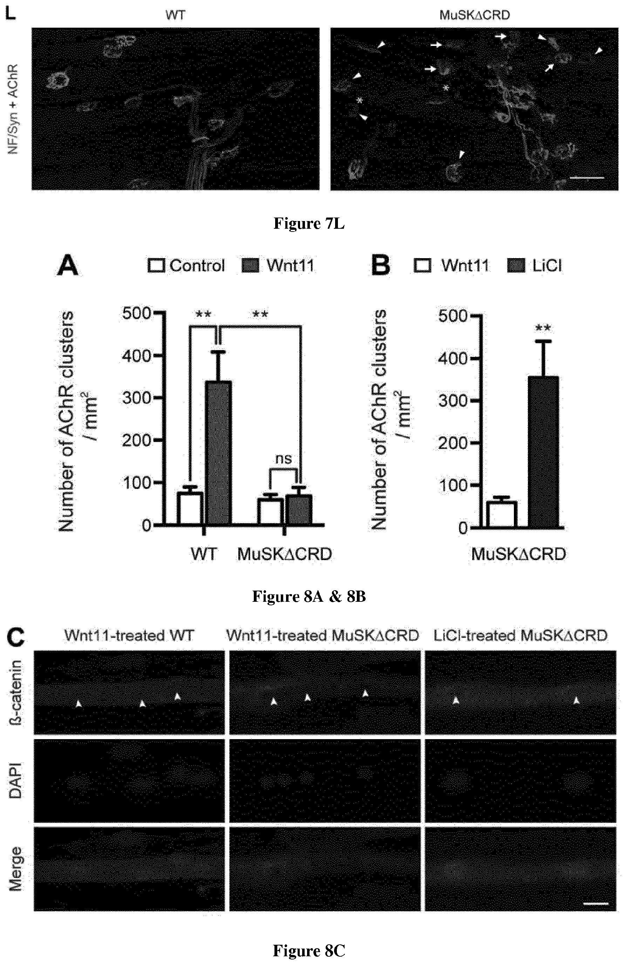

**Figure 7****. MuSKΔCRD mice progressively develop muscle weakness, fatigability and decreased muscle contraction.**
   (A) Micro-Computed tomography scans of P90 WT and MuSKΔCRD mice. (B) P120 WT and MuSKΔCRD kyphotic index. (C) Latency to fall quantifications during a rail-grip test at various time-points (P20, P40, P60 and P90). (D-E) Quantification of fore limb (D) and hind limb (E) grip strengths in WT and MuSKΔCRD mice. (F) Representative examples of twitch and tetanic contractions evoked by stimulation of the motor nerve in WT and MuSKΔCRD P120 isolated mouse hemidiaphragms. The phrenic nerve was stimulated either with single or tetanic stimuli (600 ms duration) at 20, 40, 60, 80 and 100 Hz. (G-H) Peak amplitudes of nerve-evoked single twitch and tetanic stimulations in WT and MuSKΔCRD mice. mN: millinewton ; g: gram. (I) Example of repeated tetanic nerve stimulation (60 Hz, 600 ms duration at 1 Hz) that induced a degree of fatigue more pronounced in MuSKΔCRD than in WT. (J) Quantification of the fatigability in WT and MuSKΔCRD. (K) Example of spontaneous twitch induced by unique stimulation observed in MuSKΔCRD muscles. The bottom line corresponds to the stimulator. Calibration scales in WT apply to MuSKΔCRD. (L) Confocal images of whole mount P90 WT and MuSKΔCRD left hemidiaphragms stained as in Figure 2B. Arrow, loss of postsynapse. Arrowhead, denervated postsynapse. Star, fragmented NMJ. Data are shown as mean ± SEM. *p<0.05. N = 3 animals per genotype in A-B and L, N = 6 animals per genotype in C-E, N= 5 animals per genotype in F-K. Mann-Whitney U test. Scale bar in L, 50 µm.

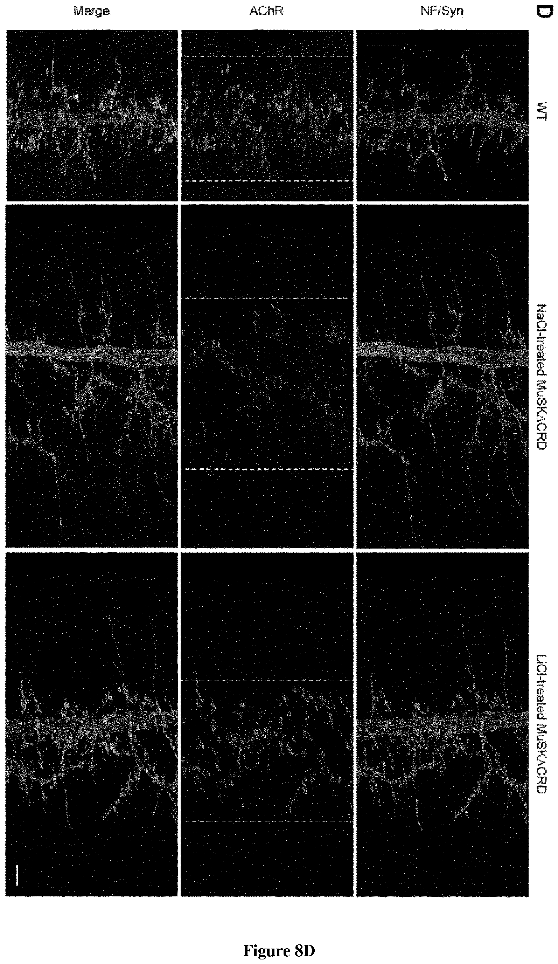

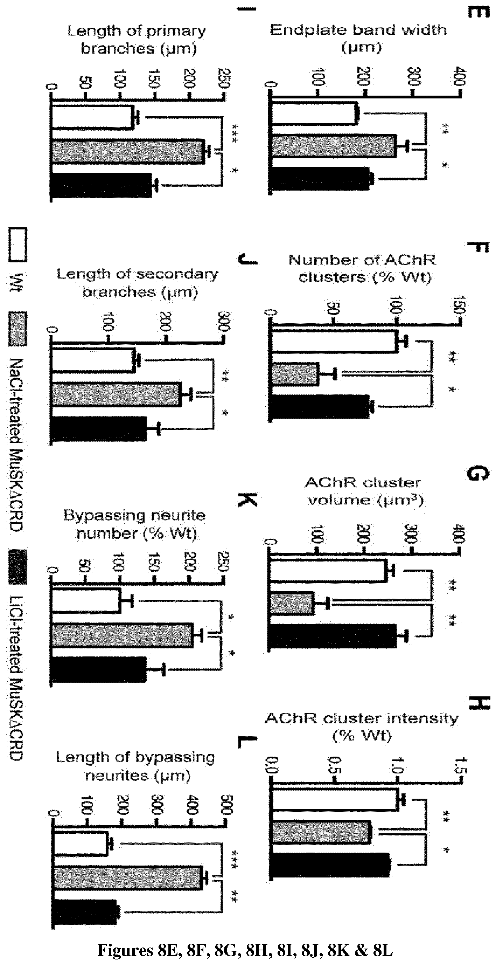

**Figure 8****. LiCl treatment rescues NMJ defects in MuSKΔCRD embryos.**
   (A-B) Quantitative analysis of the number of AChR clusters in myotubes isolated from WT or MuSKΔCRD primary cultures and treated or not with Wnt11 (A) or LiCl (B). (C) Examples of Wnt11-treated WT, Wnt11-treated MuSKΔCRD and LiCl-treated MuSKΔCRD primary myotubes stained with β□catcnin together with Dapi to visualize β□catcnin translocation to nuclei (arrowheads). (D) Confocal images of whole mount E18.5 WT, NaCl-treated MuSKΔCRD and LiCl-treated MuSKΔCRD left hemidiaphragms stained as in Figure 2B. White dashed lines delineate the synaptic endplate band and include most AChR clusters. (E-L) Quantification of the endplate band width (E), AChR clusters number (F), volume (G) and intensity (H). Number of AChR clusters tested: 2235 in WT, 846 in NaCl-treated MuSKΔCRD embryos and 1714 in LiCl-treated MuSKΔCRD embryos. (F-I) Quantitative analyses of the length of primary and secondary nerve branches (I-J), the number and the length of bypassing neurites (K-L). At least 300-400 primary and secondary nerve branches were analyzed per condition. Data are shown as mean ± SEM. *p<0.05, **p<0.01, and ***p<0.001. ns: non significant. N = 6 embryos per genotype, two-way ANOVA or Mann-Whitney U test. Scale bar in the merged image in C, 20 µm; in D, 50 µm.

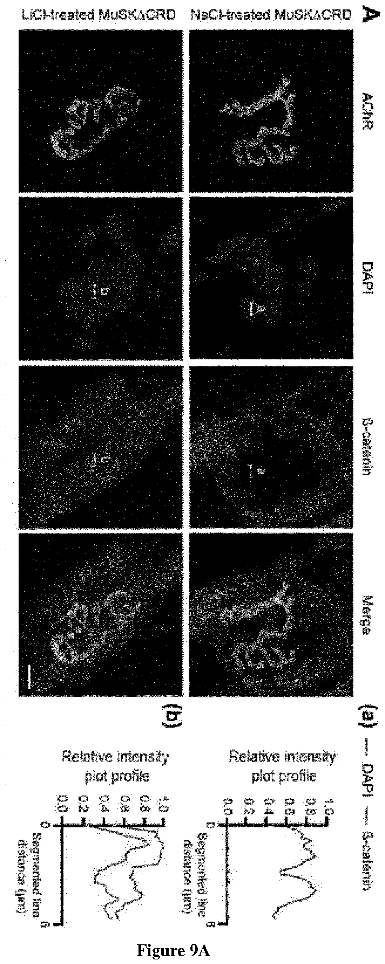

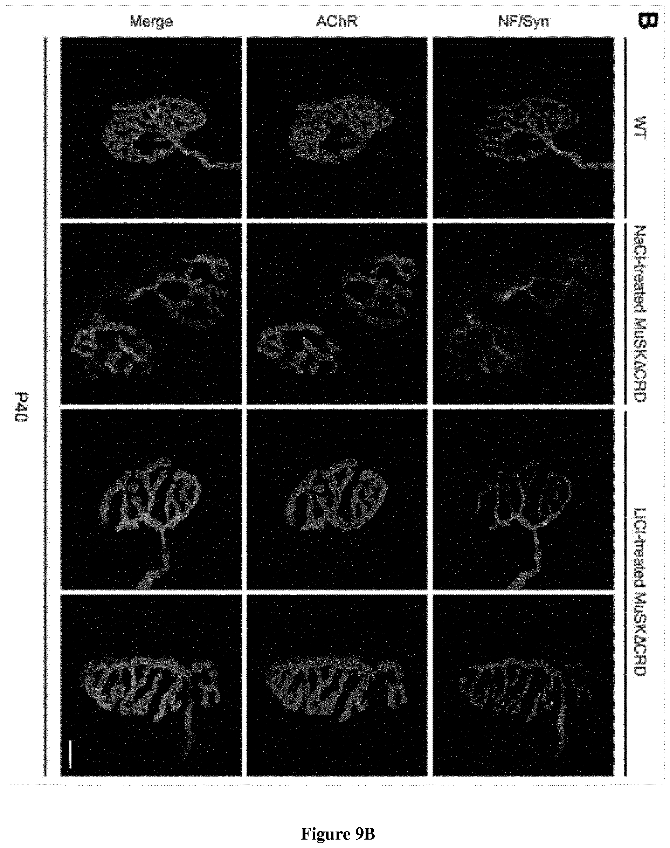

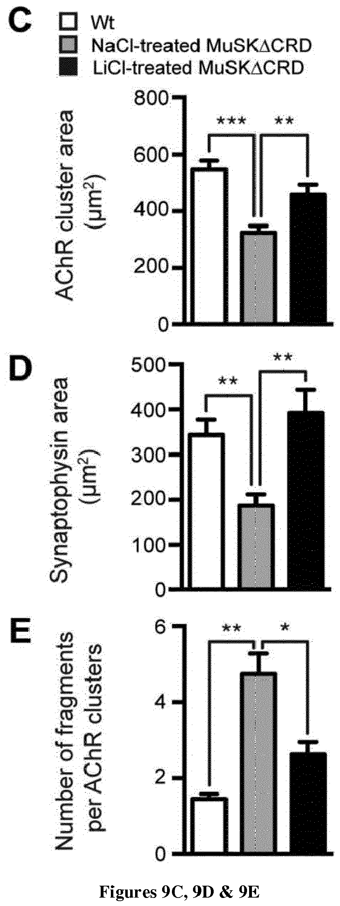

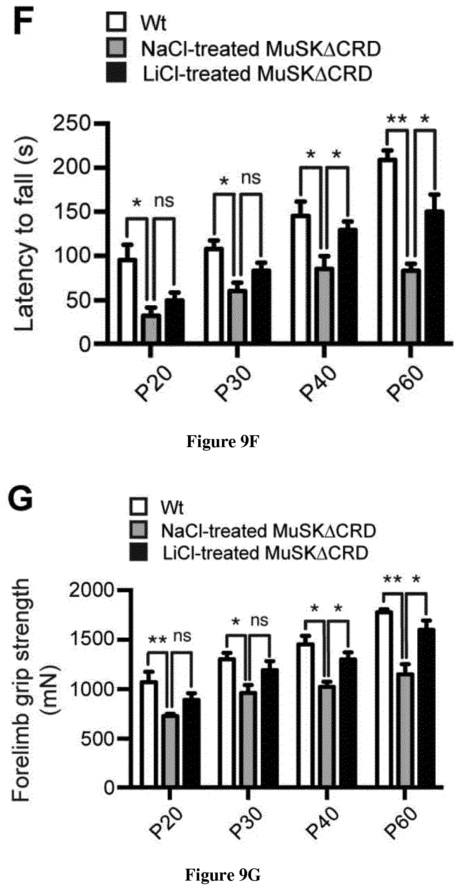

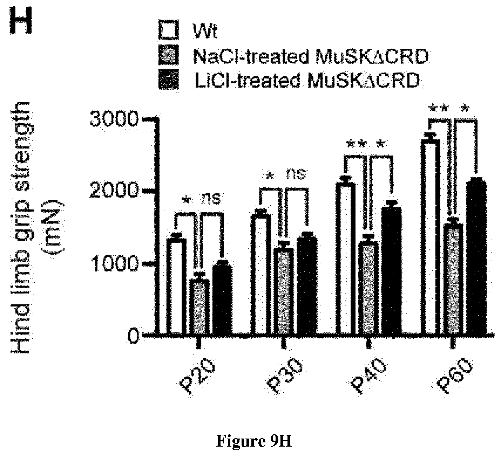

**Figure 9****. LiCl treatment restores NMJ morphological defects and motor function in adult MuSKΔCRD mice.**
   (A) Confocal images of synapses from P40 NaCl-treated or LiCl-treated MuSKΔCRD whole mount isolated TA muscle fibers stained with β-catenin (red) antibody together with α-BTX (AChRs, green) and Dapi (blue). Examples of intensity plot profiles measuring the fluorescence intensity of β-catenin and Dapi along the segmented lines corresponding to a subsynaptic nucleus are represented on the right side (a, NaCl-treated MuSKΔCRD; b, LiCl-treated MuSKΔCRD). (B) Confocal images of synapses from P40 WT, NaCl-treated or LiCl-treated MuSKΔCRD whole mount isolated TA muscle fibers stained with neurofilament (NF, red) and synaptophysin (Syn, red) antibodies together with α-BTX (AChRs, green). (C-E) Quantification analyses of the AChR cluster area (C), the synaptophysin area (D) and the number of fragments per AChR clusters (E). (F) Latency to fall quantifications during a rail-grip test at various time-points (P20, P40, P60 and P90). (G and H) Quantification of fore (G) and hind limb (H) grip strength in WT, NaCl-treated or LiCl-treated MuSKΔCRD mice. Data are shown as mean ± SEM of at least 50 NMJs. *p<0.05, **p<0.01, and ***p<0.001; ns: non significant. N = 6 animals per genotype, two-way ANOVA. Scale bar in the merged image in A and B, 10 µm.

### EXAMPLE:

### Material & Methods

### Animals

All experiments on mice were performed in accordance with European Community guidelines legislation and reviewed by the local ethical committee of the Paris Descartes University (N° CEEA34.LS.030.12). The investigators had valid licenses (N°A-75-1970) to perform experiments on live vertebrates delivered by the Direction des Services Veterinaires (Prefecture de Police, Paris, France). The animal house and the experimental room of Paris Descartes University had received the agreement of the same authority (N° B75-06-07). Experimental procedures were performed on C57BL/6 male mice and mutant mice were always compared to Wt littermates.

### Generation of MuSKΔCRD mutant mice and genotyping.

The MuSK^{ΔCRD/ΔCRD} mutant mouse line, lacking MuSK 315-478 amino acids corresponding to the CRD was established at the Mouse Clinical Institute (MCI/ICS) using proprietary vector containing floxed Neomycin resistance cassette and Protamine-Cre cassette (Illkirch, France; http://www-mci.u-strasbg.fr). The use of protamine cassette in the construction vector offers an efficient solution for auto-excision of the floxed region when chimaera mice were bred with Cre-expressing mice. The targeting vector was constructed by successive cloning of PCR products and contained a 5.5 kb fragment (corresponding to the 5' homology arm), a 4 kb floxed fragment including Protamine Cre and Neomycin selection cassettes, and a 5 kb fragment (corresponding to the 3' homology arms). Two LoxP sequences delimitating the floxed fragment were located upstream of exon 9 and downstream of exon 11. The linearized construct was electroporated in Balb/CN mouse embryonic stem (ES) cells. Targeted ES clones were screened by 5' external and Protamine Cre (inside the targeting vector) LongRange PCR and by Neo Southern blot. Two positive ES clones were injected into C57BL/6N blastocysts, and derived male chimaeras gave germline transmission. The resulting lines were crossed with a Cre deleter mouse generated on a pure inbred C57BL/6N background, in which the CRE gene is driven by the chicken β actin promoter, and showing high and stable recombination efficiency to induce deletion of the floxed region (Birling et al., 2012). Forword (Ef) and reverse (Wr and Lxr) primers were used for genotyping for the WT allele and for the MuSKΔCRD allele. The WT amplified sequence was 234 bp-long while the knock-out amplification was 200 bp-long.

### Antibodies

The following antibodies were used: polyclonal and monoclonal Alexa Fluor® 488 conjugated (Life Technologies, 1/1000), polyclonal Cy™ 3-conjugated (Jackson immunoresearch, 1/1000), monoclonal and polyclonal Peroxidase conjugated (Amersham, 1/10000), rabbit monoclonal anti-synaptophysin (Life Technologies, 1/5), polyclonal anti-neurofilament 68 kDa (Chemicon, 1/1000), polyclonal anti-neurofilament 165 kDa (DSHB, Iowa, USA, 1/750), polyclonal anti-HA (1/2500; Abcam), monoclonal anti-beta catenin (Life Technologies, 1/500). Polyclonal anti-MuSK (Abcam, 1/200), monoclonal anti-transferrin receptor (TfR, Invitrogen, 1/500) and anti-α-tubulin (Sigma-Aldrich, 1/6000) were used for western blot. Polyclonal anti-MuSK (1/500) used for immunohistochemistry is a gift from M. Ruegg (Germany). Dapi (1/20000) and α-bungarotoxin (α-BTX) Alexa Fluor® 488 conjugate (1/1000) were purchased from Euromedex and Life Technologies, respectively.

### Plasmids

The rat MuSK-HA and MuSKΔCRD cDNA plasmids have been previously described (Cartaud et al., 2004; Strochlic et al., 2012).

### Biotinylation of cell surface MuSK and MuSKΔCRD and western blot analyses

HEK293T cells (ATCC) were cultured in DMEM supplemented with 10% fetal bovine serum, 2 mM glutamine and 2%penicillin/streptomycin (500U) at 37°C in 5% C0₂. Cells were grown to 70% confluence and transfected (2 to 7 µg of plasmids) using Fugen (Promega) transfection technique. 48h after transfection, cells were washed with cold PBS containing 1mM MgCl2 and 0.1 mM CaCl2 and incubated with 0.5 mg/ml EZ-link NHS-SS-biotin (Thermo Scientific Pierce) in the same buffer at 4°C for 30 min. The labeling reaction was quenched by incubation with 50 mM glycine and 0.5% BSA for 5 min. Cells were then rinsed and harvested in lysis buffer (Tris 50 mM, NaCl 150 mM, EDTA 3mM, Triton X100 1%) containing a cocktail of protease inhibitors (Roche). Lysates were centrifuged at 20000g for 15 min and supernatants were pre-cleared with Sepharose beads. Biotin-labeled proteins were recovered by incubation with BSA-treated streptavidin-agarose beads for 3h at 4°C (Thermo Scientific Pierce). Bound proteins were resolved by a 7% NuPAGE Novex Tris-acetate gel and detected by Western blot using HA antibodies. The membrane transferrin receptor (TfR) was used as a loading control to normalize the results. Relative signal intensity of total and cell surface MuSK or MuSKΔCRD proteins was measured using ImageJ software. The levels of MuSK and MuSKΔCRD in total extracts were normalized to tubulin-alpha signals.

### Muscle primary cultures.

Muscle cells were isolated from P7-P10 hind limb Tibialis Anterior (TA) and Gastrocnemius muscles from MuSΔCRD or WT mice. Briefly, muscle tissues were excised, separated from connective tissue, minced in dissecting medium (DMEM-F12 medium containing 2 mM glutamine, 2%penicillin/streptomycin (500U), 2% Fungizone) and dissociated in dissecting medium containing 0.2% type I collagenase (Gibco) for 90min at 37°C in water bath. Cells were centrifugated, filtered and resuspended in proliferating medium (dissecting medium supplemented with 20% horse serum and 2% Ultroser G, Pall). After overnight pre-plating, cells were expanded in Matrigel (Corning) coated dishes for 3 to 5 days and differentiated in differentiating medium (dissecting medium supplemented with 2% horse serum) for 5 days. When indicated, myotubes were treated with recombinant Agrin (0.4µg/ml, R&D system), Wnt11 (10ng/ml, R&D system) or LiCl (2.5 mM, Sigma) for 16h.

### Computed Tomography scan analysis and measurement of the kyphotic index.

Micro Computed Tomography (micro-CT) scan analysis was performed in collaboration with the imaging platform PIPA installed in the imaging laboratory of EA 2496 (Montrouge). P90 WT and MuSKΔCRD mice were sedated using 1.5% isoflurane in air (TEC 3 Anestéo France). Entire body in dorsal and ventral decubitus of each animal was scanned by Quantum FX Perkin Elmer micro-CT device (Caliper Rikagu) in dynamic mode. A tube voltage of 90 kV and a tube current of 160 µA were selected. Total scan time was 2x17 seconds per total animal scan. The scan field of view was 2x60 mm with a spatial resolution of 118 micros (voxel size). Each scan was achieved by optimizing the resolution/dose ratio. Therefore, for the resolution selected, each animal was exposed to a low dose of 26 mGy. Image reconstructions and measurements were performed by the Osirix software v.5.6. The kyphotic index (KI) was determined from the direct multiplanar reconstructions as previously described (Laws and Hoey, 2004). Briefly, the distance (D1) from the seventh cervical vertebra (C7) to the sixth lumbar vertebra (L6) and then the perpendicular distance (D2) from D1 to the point of maximum vertebra curvature were measured. KI corresponds to the ratio D1/D2. KI is inversely proportional to kyphosis.

### Grip strength measurement

Animals were allowed to grasp a metal rail suspended in midair and their latency to release the rail was recorded. Each mouse (N=6 for each genotype) was subjected to five trials with at least 10 min rest period between tests.

The grip strength was measured using a grip force tensiometer (Bioseb) according to the TREAT-NMD guidelines. Forelimbs and hindlimbs traction strength were recorded according to the manufacter's instructions. Three measurements were performed per animal.

### Ex vivo isometric tension analyses

P120 Wt and MuSKΔCRD mice were euthanized by dislocation of the cervical vertebrae followed by immediate exsanguination. Left hemidiaphragm muscles with their respective associated phrenic nerves, were mounted in a silicone-lined bath filled with Krebs-Ringer solution of the following composition: 154 mM NaCl, 5 mM KCl, 2 mM CaCl2, 1mM MgCl2, 11mM glucose and 5 mM HEPES (buffered at pH 7.4 with NaOH), continuously perfused with O2 at 23.1+/- 0.4°C. One of the hemidiaphragm tendons (at the rib side) was securely anchored onto the silicone-coated bath via stainless steel pins, while the other tendon was tied with silk thread, via an adjustable stainless steel hook, to an FT03 isometric force transducer (Grass Instruments, West Warwick, RI, USA). Muscle twitches and tetanic contractions were evoked by stimulating the motor nerve via a suction micro electrode adapted to the diameter of the nerve, with supramaximal current pulses of 0.15 ms duration, at frequencies indicated in the text. For each preparation investigated, the resting tension was adjusted at the beginning of the experiment (to obtain maximal contractile responses), and was monitored during the whole duration of the experiment. Signals from the isometric transducer were amplified, collected and digitized with the aid of a computer equipped with an analogue to digital interface board (Digidata 1200, Axon Instruments, Union City, CA, USA) using Axoscope 9 software (Axon Instruments).

### Electron microscopy

P120 Wt and MuSKΔCRD mice were sedated using 1.5% isoflurane in air (Minerve Equipement veterinaire). Tibialis anterior were then dissected and immediately fixed in 2% glutaraldehyde and 2% paraformaldehyde in PBS for 1 h at room temperature and overnight at 4°C. TA muscle were rinsed in PBS and AChE staining following Koelle's protocol was performed. The endplate-containing tissue blocks were cut in small pieces. Subsequently, tissue samples were washed three times in 0.1M sodium phosphate buffer, incubated 30 min at 4°C in 1% osmic acid in sodium phosphate buffer and dehydrated in graded ethanol solutions (2x10 min in 70%, 2x10 min in 90% and 2x10 min in 100% ethanol). Samples were then incubated twice in propylene for 1 min, 10 min in 50% 50% Epon-50% propylene oxide, then embedded in Epon and incubated for polymerisation 24h at 60°C. Finally, 90 nm sections were cut on a Reichert Ultracut S and layed on a grid for staining 10 min with 2% uranyl acetate and 4 min with lead citrate at RT. The observations were performed with a JEOL 1011 transmission electron microscope and the images recorded at 80 kv with a GATAN Erlangshen 1000 camera.

### Whole mount staining of diaphragms

Diaphragm muscles were dissected and fixed (4% paraformaldehyde in PBS) for 1 h at room temperature and further fixed (1% formaldehyde in PBS) overnight at 4°C. Muscles were washed three times for 15 minutes in PBS, incubated for 15 minutes with 100 mM glycine in PBS and rinsed in PBS. Muscles were permeabilized (0.5% Triton X-100 in PBS) for 1 h and blocked for 4 hours in blocking buffer (3% BSA, 5% goat serum and 0,5% Triton X-100 in PBS). Muscles were incubated overnight at 4°C with rabbit polyclonal antibodies against neurofilament and synaptophysin in blocking solution. After three 1-hour washes in PBS, muscles were incubated overnight at 4°C with Alexa-488 goat anti-rabbit IgG and Alexa-594-conjugated-a-bungarotoxin (α-BGT) in blocking solution. After three 1-hour washes in PBS, muscles were flat-mounted in Vectashield (Vector Labs) mounting medium.

### Immunohistochemistry (tissue sections and isolated muscle fibers)

For tissue sections analyses, dissected *Soleus* and Tibialis Anterior muscles from P90 adult mice were fixed (1% paraformaldehyde in PBS) for 1 hour at 4°C, rinsed twice at 4°C in PBS, cryoprotected (30% sucrose in PBS) overnight at 4°C, embedded in TissueTek (Sakura) and quickly froze in isopentane cooled in liquid nitrogen. Cryostat cross sections (12 µm) were permeabilized with 0.5% Triton X-100 in PBS for 10 min and labeled with various antibodies as for whole mount immunostaining. Incubation times were the following: 1 hour for blocking, overnight at 4°C for incubation with primary antibodies and 1 hour for incubation with secondary antibodies.

For isolated muscle fibers analyses, dissected and isolated *Soleus, Extensor Digitorum Longus* (EDL) and Tibialis Anterior muscles fibers from P20 and P60 adult mice were fixed (4% paraformaldehyde in PBS) for 30 min at 4°C and rinsed with PBS at RT. Isolated muscle fibers were labeled with antibodies as for whole-mount immunostraining. Incubation times were the following: 3 hours for blocking, overnight at 4°C for incubation with primary or secondary antibodies.

### Images acquisition and processing

All images were collected on a microscope (model BX61; Olympus) equipped with a Fast 1394 Digital CCD FireWire camera (model Retiga 2000R; Qimaging) and a 20X objective or on a confocal laser scanning microscope (Zeiss LSM-710) equipped with a 20X objective and 63X oil objective. Collected Z-stacks confocal images (5 to 20 stacks with 1 to 1,5 µm (20X) z-steps) and image capture were made using LSM Image Browser. The same laser power and parameter setting were applied to ensure fair comparison between WT and MuSKΔCRD muscles. Multiple Tile Scanned images were taken to cover the size of the whole diaphragm. Confocal images presented are single-projected image derived from overlaying each set of stacks. For quantification of the AChR clusters number, volume and intensity, image stacks were quantified using the ImageJ (version 1.46m) plugin "3D object counter" (Bolte and Cordelières, 2006). The threshold intensity was set by visual inspection of AChR clusters, being the same between WT and MuSKΔCRD images. The endplate band width was defined by the distance between the two farthest AChR clusters from the main nerve trunk. Around 100 measurements regularly spaced and covering the entire diaphragm were taken. At least 4 diaphragms or 50 isolated muscle fibers of each genotype were analyzed and quantified. To evaluate β-catenin translocation to subsynaptic nuclei in isolated muscle fibers, image stacks corresponding to nuclei were used for quantification using the ImageJ intensity plot profile measuring the intensity of β-catenin and Dapi fluorescence within the segmented line.

### Neonatal and pregnant mice intra-peritoneal injections.

E12 pregnant mice or P10 adult mice were intraperitoneally injected with LiCl (Sigma, 600mM-10µl/g body weight) or placebo NaCl solution (0,0009%-10µl/g body weight). Daily injections were made from E12 to E18 or from P10 to P60. After embryos genotyping, E18.5 or P60 diaphragms of LiCl-treated MuSKΔCRD mice were analyzed and compared to WT or NaCl-treated MuSKΔCRD.

### Statistical Analysis

All data were expressed as means ± SEM. Statistical analyses and graphs were performed with Prism 6.0 (Graphpad) software. All data were analyzed using the Mann-Whitney U-test or two-way ANOVA, wherever appropriate (P<0.05 considered significant). Each experiment was conducted a minimum of three times.

### Results

### Generation of MuSKΔCRD transgenic mice.

The inventors generated a mouse line deleted from the MuSK CRD by homologous recombination (Figure 1A). To construct the MuSK-CRD floxed allele, we created a targeting vector consisting of the MuSK gene flanked by two loxP sites upstream of exon 9 and downstream of exon 11. The construct was electroporated in Balb/CN mouse embryonic stem (ES) cells and the injected clones were controlled by Neo Southern blot performed on two distinct digests using a Neo probe to verify the 5' arm integration by homologous recombination (Apa L1 and Eco R1) and the 3' arm integration (Drd 1 and Xcm 1, Figure 1B). For all digests, a single band of the expected size was obtained indicating correct integration and absence of second random integration. Two positive and independent ES cell clones were injected into C57BL/6N blastocysts to generate two independent MuSK^{flox(CRD)} mice. These mice were then crossed with Cre deleter mice in order to generate offspring with MuSK CRD deletion (see Material and Methods, Figure 1C). Moreover, a single band corresponding to MuSK deleted from its CRD was detected in MuSKΔCRD primary myotubes after MuSK immunoprecipitation confirming that the CRD deletion occurred in the mutant mice (Figure 1D). Both heterozygous MuSK^{+/ΔCRD} and homozygous MuSK^{ΔCRD/ΔCRD} (MuSKΔCRD) mutant mice were indistinguishable from wild-type (WT) mice. Although 5 % of the MuSKΔCRD mice died few days after birth being smaller in weight and size, exhibiting respiratory failure as well as limb motor deficiency, most of them were viable, able to suck milk and developed up to adulthood with normal fertility. In addition, no difference in the weight curve between MuSKΔCRD and WT mice could be detected.

Given that deletion of MuSK CRD could lead to a deficit of the mutated MuSK expression at the cell membrane, we quantified the level of membrane WT or mutated MuSK using in vitro biotinylation experiments and tissue immunohistochemistry. Labeling of surface proteins by biotinylation in HEK293T cells expressing MuSK-HA or MuSKΔCRD-HA showed similar levels of MuSK and MuSKΔCRD at the plasma membrane (Figure 1E). Moreover, both WT and mutated MuSK colocalized with membrane AChRs clusters labeled with α-bungarotoxin (BTX) in P60 WT and MuSKΔCRD tibialis anterior (TA), indicating that deletion of MuSK CRD does not disrupt the mutated MuSK membrane expression at the NMJ (Figure 1F). Quantification of the level of WT and mutated MuSK signal intensity at the synapse, showed an increase of 70% in MuSKΔCRD compared to WT mice (Figure 1G). In addition, since MuSK localization at the synapse is required for anchoring acetylcholinesterase (AChE) in the postsynaptic membrane (Cartaud et al., 2004), we asked whether AChE localization and activity was disturbed in MuSKΔCRD muscles. Histochemical staining revealed that AChE was accumulated in the postsynaptic membrane of both WT and MuSKΔCRD P90 TA muscles (Figure 1H). No difference in AChE activity could be detected between WT and MuSKΔCRD muscles (Figure 1I).

To test whether deletion of MuSK CRD could disturb agrin-Lrp4-MuSK signaling, we quantified agrin-induced AChR clusters in WT and MuSKΔCRD primary muscle cultures. No difference in the number of AChR clusters following agrin treatment was detected in MuSKΔCRD compared to WT primary myotubes, indicating that MuSK CRD deletion does not perturb agrin-induced AChR clustering. These data suggest that agrin is able to interact with Lrp4 in the Lrp4/MuSKΔCRD complex and that MuSKΔCRD/Lrp4 interaction can transduce downstream signaling (Figure 1J and 1K).

### Deletion of MuSK CRD affects NMJ formation.

Given that Wnts are known to play a role during the early steps of NMJ formation through binding to MuSK CRD (Jing et al., 2009; Strochlic et al., 2012), we tested whether deletion of MuSK CRD could lead to NMJ defects during development. First, we compared NMJ phenotype in E14 WT and MuSKΔCRD embryos (Figure 2). Whole-mount diaphragms were stained with α-BTX to detect AChR clusters and with a mixture of antibodies against neurofilament (NF) and synaptophysin (Syn) to label axonal branches and nerve terminals respectively. Both dorsal and ventral portions of each hemidiaphragm were innervated indicating that axonal extension is fully developed in MuSKΔCRD embryos (Figure 2A). Nonetheless, the neurites were increased in length by 82% (Figure 2B and 2C). In addition to this presynaptic defect, AChR clustering was also affected in MuSKΔCRD embryos. In WT embryos, AChR clusters were concentrated in the central zone of the muscle as expected during prepatterning. In contrast, in mutant embryos, AChR clusters were almost undetectable and were distributed in a 2-fold wider muscle area (Figure 2B and 2D, arrowheads). Quantitative analysis revealed a mean 64% decrease in the number of MuSKΔCRD AChR clusters (Figure 2E). Moreover, MuSKΔCRD AChR clusters volume and intensity were respectively reduced by 70% and 65% and non innervated AChR clusters were increased by 30% in MuSKΔCRD compared to WT embryos (Figures 2F-2H). These results suggest that deletion of MuSK CRD leads to an early developmental defect of the NMJ with a severely reduced AChR prepatterning.

We further analyzed NMJ morphology later during development in E18.5 MuSKΔCRD diaphragms (Figure 3). Whereas AChR clusters were tightly restricted to a thin endplate band in WT hemidiaphragms, the endplate band width was 2-fold larger in MuSKΔCRD (Figures 3A and 3B). Furthermore, AChR clusters were reduced in number by 40% (Figure 3C), volume by 25% (Figure 3D), and intensity by 14% (Figure 3E). All AChRs clusters were innervated in WT and MuSKΔCRD, however, MuSKΔCRD embryos showed aberrant extension of motor axons, bypassing AChR clusters and growing excessively toward the periphery of the muscle (Figure 3F). Although the number of primary and secondary branches was not significantly affected, the length of primary as well as secondary branches was increased by 75% and 46% respectively in MuSKΔCRD mutants compared to WT embryos (Figures 3G-J).

Taken together, these results indicate that MuSK CRD deletion drastically perturbs NMJ formation as exemplified by reduced prepattemed and neural AChR clusters as well as exuberant neurite outgrowth.

### MuSKΔCRD adult mice exhibit immature and fragmented NMJs.

As previously mentioned, MuSKΔCRD mutant mice are viable at birth. Therefore, we wondered whether NMJ defects observed during development would be also detected in newborn and adult mutant mice. NMJ phenotype analyzed in P5 WT and MuSKΔCRD whole-mount preparations of diaphragm revealed AChR clusters deficit and neurite outgrowth defects in MuSKΔCRD similar to those observed in MuSKΔCRD embryos (Figure 4A-E). To assess whether the pre- and postsynaptic counterparts were still affected in MuSKΔCRD adult mice, we analyzed NMJ morphology on isolated muscle fibers from WT and MuSKΔCRD TA muscles at P20 and P60 (Figure 5). At birth, the shape of the endplates is ovoid and as NMJs mature, the endplates begin to acquire their branched postnatal topology (Kummer et al., 2006; Marques et al., 2000). In P20 MuSKΔCRD mice, most of the endplates analyzed were ovoid and compact compared to the perforated WT ones suggesting that NMJs are immature (Figure 5A, left panel). Both analyzed AChR endplates of WT and MuSKΔCRD mice were innervated as confirmed by nerve terminals staining which colocalized with AChR clusters (Figure 5A, left panel). However, quantitative analysis revealed that the number of AChR clusters as well as nerve terminal area per NMJ were significantly reduced by 49% and 58% respectively in MuSKΔCRD mice compared to WT mice without affecting the final overlap area between pre and postsynaptic elements (Figure 5B-D).

At P60, WT NMJs formed a continuous branched postnatal topology and exhibited a typical "pretzel-like" structure (Figure 5A, right panel). At this stage, 10% of the analyzed NMJs in MuSKΔCRD were similar in shape to WT ones. However, the structure of most MuSKΔCRD synapses (90% of the analyzed NMJs) was severely altered with the following characteristics: the postsynaptic network was discontinuous and isolated AChR clusters were frequently observed, suggesting fragmentation of the NMJs. Indeed, the number of AChRs fragments per NMJ was increased by 4-fold (Figure 5E). Moreover, the total occupied AChR clusters area per NMJ was significantly reduced by 40%, suggesting a loss of AChR-rich domains (Figure 5F). Axonal branches appeared discontinuous and fragmented in correlation with the postsynaptic apparatus parceling. The nerve terminal area was reduced by 39% without affecting the overlap area between pre and postsynaptic elements (Figure 5G and 5H). Similar NMJ defects were observed in other muscles types including fast-twitch extensor digitorum longus (EDL) and slow-twitch soleus.

Taken together, our findings indicate that the CRD deletion of MuSK perturbs NMJs maturation that finally leads to a severe dismantlement of the post- and presynaptic apparatus in adult mice.

### MuSKΔCRD mice display altered NMJ ultrastructures.

To analyze the morphological alterations of muscle and NMJs at the ultrastructural level in MuSKΔCRD mice, we performed electron microscopic analysis on TA muscle of P120 WT and MuSKΔCRD mice (Figure 6A-M). Although presynaptic specialization of nerve terminals appeared normal in MuSKΔCRD mice with abundant mitochondria in the axoplasm, quantification analyses revealed a 67% decrease in MuSKΔCRD synaptic vesicle density compared to WT mice without affecting the mean synaptic vesicle diameter (Figure 6H and 6I). Moreover, on the postsynaptic side, about 30% of MuSKΔCRD NMJs exhibited highly disorganized or very few junctional folds (JFs) compared to WT mice (78% decrease of JFs in MuSKΔCRD, Figure 6A-F and 6J). In some case, compared to WT, the distance between JFs and muscle fibers was increased and abundant accumulation of mitochondria beneath the postsynaptic membrane was observed in MuSKΔCRD mice (Figure 6G). However, no morphological difference between WT and MuSKΔCRD overall muscle structure could be detected (Figure 6K). Indeed, the sarcomeric organization and the distance between Z-lines were similar between WT and MuSKΔCRD mice (Figure 6L). In addition, MuSKΔCRD mice did not show any alteration in the myelin sheath diameter (Figure 6M).

Collectively, these results indicate that MuSK CRD deletion alters both pre- and postsynaptic ultrastructures in MuSKΔCRD NMJs, which may result in a motor activity deficit.

### MuSKΔCRD mice progressively develop muscle weakness, fatigue and motor defects.

During the first two weeks after birth, MuSKΔCRD mice developed normally without any gross physical signs of muscle weakness compared to WT mice. After this period, changes in the trunk region became progressively evident by the abnormal spine curvature caused by the shrinkage of the pelvic and scapular region. The computed tomography scan analysis performed on P90 WT and MuSKΔCRD mice illustrates the kyphosis developed by MuSKΔCRD mice (Figure 7A). The kyphotic index (KI, see Material and Methods) used to appreciate the kyphotic severity degree, was significantly reduced in MuSKΔCRD compared to WT confirming the presence of a severe spine deformation in mutant mice (Figure 7B, Laws and Hoey, 2004). To assess whether motor function was altered in MuSKΔCRD mice, we performed a grip test assay on young and adult mice. Whereas the latency to fall from the rail increased with age in WT mice, MuSKΔCRD mice exhibited poor motor performance from P20 to P90 as determined by a reduced latency to fall (latency decrease: P20, 62%; P40, 33%; P60, 64%; P90, 72%; Figure 7C). To further investigate the origin of motor defect in MuSKΔCRD mice, we measured the grip strength of the forelimbs (Figure 7D) and the hind limbs (Figure 7E). In MuSKΔCRD as in WT mice, the forelimb and the hind limb grip strength increased with age. However, the grip strength of MuSKΔCRD mice was reduced at all time-points compared to WT mice indicating a muscle weakness (forelimb grip strength decrease: P20, 26%; P40, 23%; P60, 20%; P90, 30%; hindlimb grip strength decrease: P20, 37%; P40, 18%; P60, 23%; P90, 21%; Figures 7D and 7E).

To confirm the occurrence of muscle weakness in MuSKΔCRD, we analyzed the ability of P120 WT and MuSKΔCRD left hemidiaphragms to evoke twitches and tetanic contractions in response to phrenic nerve stimulation at different frequencies ex vivo. As shown in Figure 7F, WT as MuSKΔCRD muscles developed and maintained tetanic contractions, in a wide range of nerve-stimulation frequencies. However, MuSKΔCRD muscles developed less force than WT ones. Indeed, in MuSKΔCRD mice, the strength of muscle twitch upon nerve stimulation was significantly reduced compared to WT mice, both upon single and tetanic stimulations (strength decrease of muscle twitch: single twitch, 59%; T40 Hz, 54%; T60 Hz, 47%; T80 Hz, 44%; T100 Hz, 42%; Figure 7G). The developed muscle specific force, defined as the muscle strength (mN) normalized to the muscle weight (g) was significantly reduced in MuSKΔCRD compared to WT muscles, both upon single and tetanic (T100 Hz) stimulations respectively by 52% and 24% (Figure 7H). One expected pathophysiological consequence of NMJ structural changes is fatigable muscle weakness as observed in myasthenia (Hantaï et al., 2013). We therefore evaluated the muscle fatigue strength after a train of tetanic nerve stimulations (T60 Hz) and found that MuSKΔCRD muscles exhibited a degree of fatigue more pronounced than WT muscles (fatigability increase: 30%, Figure 7I and 7J). Interestingly, we also observed spontaneous twitches after one twitch induced by unique stimulation in MuSKΔCRD mice, suggesting the presence of muscle denervation processes (Figure 7K, Heckmann and Ludin, 1982). To further confirm this issue, non innervated AChR clusters could be detected in P90 MuSKΔCRD whole-mount diaphragm preparations (Figure 7L).

Our data demonstrate that MuSK CRD deletion compromises motor performance, affects muscle strength and lead to increased muscle fatigability. This is in agreement with clinical symptoms generally observed in patients suffering from CMS.

### Lithium chloride rescues NMJ phenotype of MuSKΔCRD mice.

Deletion of MuSK CRD, impairing Wnt/MuSK interaction is likely to perturb Wnt signaling at the NMJ. Indeed, treatment of WT primary myotubes with Wnt11, a member of the Wnt family known to interact with MuSK CRD and required for AChR clustering (Jing et al., 2009; Zhang et al., 2012) induced a 4.5-fold increase in the number of AChR clusters that was fully abolished (80% decrease) in Wnt11-treated MuSKΔCRD primary myotubes, demonstrating that deletion of MuSK CRD alters Wnt-induced AChR clustering (Figure 8A). In addition, β-catenin translocation to the nucleus was strongly reduced in Wnt11-treated MuSKΔCRD compared to Wnt11-treated WT primary myotubes, indicating that Wnt activation of the canonical pathway is affected in MuSKΔCRD muscle cells (Figure 8C). Since previous reports suggest that the Wnt canonical pathway is involved in neuromuscular synapse formation (Li et al., 2008; Liu et al., 2012; Wu et al., 2012), we then reasoned that forced activation of the Wnt beta-catenin signaling pathway during development could thwart impaired NMJ formation and compensate at least partially MuSKΔCRD NMJ phenotype. To test this hypothesis, we set up a pharmacological approach using LiCl, a well-known reversible inhibitor of the Gsk3 and activator of Wnt/β-catenin signaling (Klein and Melton, 1996; Stambolic et al., 1996; Wada, 2009). LiCl treatment of MuSKΔCRD primary myotubes resulted in a 6-fold increase in the number of AChR clusters and increased β-catenin translocation to the nucleus compared to Wnt11-treated MuSKΔCRD myotubes suggesting that LiCl is able to rescue AChR clustering and Wnt canonical signaling in MuSKΔCRD myotubes (Figure 8B and 8C). We then tested the effect of LiCl treatment on NMJ formation in vivo in MuSKΔCRD mice. Repeated intraperitoneal injections of LiCl or placebo (NaCl) from E12 to E18.5 in pregnant mice were performed and the phenotype of E18.5 LiCl-treated MuSKΔCRD NMJs was compared to NaCl-treated MuSKΔCRD and WT NMJs (Figure 8D). Remarkably, LiCl treatment almost fully rescued the postsynaptic phenotype in E18.5 MuSKΔCRD embryos (Figure 8D). The endplate band width in LiCl-treated MuSKΔCRD embryos was reduced by 23% compared to NaCl-treated mutant embryos and was close to the endplate band of WT embryos (Figure 8E). Moreover, LiCl-treated MuSKΔCRD embryos significantly gained AChR clusters in number (by 103%), volume (by 186%) and intensity (by 20%) and were almost indistinguishable from WT embryos (Figure 8F-H). In addition, presynaptic defects were improved in LiCl-treated MuSKΔCRD. The increased length of primary and secondary branches observed in MuSKΔCRD was reduced respectively by 35% and 28% in LiCl-treated MuSKΔCRD embryos, being almost similar to WT embryos (Figure 8I and 8J). In addition, the number and length of bypassing neurites were reduced by 33% and 138% respectively in LiCl-treated MuSKΔCRD embryos compared to NaCl-treated ones (Figure 8K and 8L). Taken together, these results indicate that LiCl treatment almost fully rescued both pre- and postsynaptic defects of MuSKΔCRD mutants, with NMJs being phenotypically indistinguishable from WT NMJs.

To further investigate the beneficial effect of LiCl treatment on NMJ maintenance in adulthood, MuSKΔCRD mice were injected with LiCl or placebo (NaCl) from P10 to P60 and NMJ morphology and motor functions as well as beta-catenin translocation to subsynaptic nuclei were analyzed (Figure 9). LiCl treatment resulted in a strong increase of β-catenin translocation to subsynaptic nuclei in LiCl-treated MuSKΔCRD compared to NaCl-treated MuSKΔCRD isolated P40 TA muscle fibers as shown in the intensity plot profiles measuring the fluorescence intensity of beta-catenin and Dapi along the segmented lines (Figure 9A). In addition, we found that NMJ structures of P40 TA isolated muscle fibers from LiCl-treated MuSKΔCRD were increased in size compared to NaCl-treated MuSKΔCRD NMJ (Figure 9B). The AChR cluster and synaptophysin area in LiCl-treated mutants were increased by 40% and 105%, respectively compared to NaCl-treated mice (Figure 9C and 9D). Importantly, the number of AChRs fragments per NMJ in LiCl-treated mutants was decreased by 44% compared to NaCl-treated mice (Figure 9E). Moreover, LiCl treatment significantly improved MuSKΔCRD mice latencies to fall from the rail grip as well as fore and hind limb strength compared to NaCl-treated MuSKΔCRD mice (Figure 9F-H). Taken together these data demonstrate that LiCl treatment to postnatal MuSKΔCRD mice improves the NMJ morphological defects, muscle strength and restores beta-catenin translocation to synaptic nuclei suggesting that MuSK CRD plays a role during NMJ maintenance in adulthood likely in part via activation of the Wnt β-catenin signaling pathway

### Discussion:

Here, we have investigated the functional role of the MuSK-Wnt binding domain (CRD) during NMJ formation and maintenance in vivo. To this end, we generated mutant mice deficient for MuSK CRD. Deletion of MuSK CRD leads to severe alteration of both pre- and postsynaptic elements during early muscle prepatterning (E14) and NMJ differentiation (E18.5) mainly characterized by (i) a drastic deficit in AChR clusters and (ii) exuberant axonal growth bypassing AChR clusters. Moreover, MuSK CRD deletion is pathogenic in adult mice, inducing CMS-like symptoms including kyphosis, NMJs dismantlement, muscle weakness and fatigability as previously observed in other mice models of CMS (Bogdanik and Burgess, 2011; Chevessier et al., 2008, 2012; Gomez et al., 1997; Webster et al., 2013). We also report that NMJ innervation defects in MuSKΔCRD mice can be rescued in vivo by LiCl treatment. Taken together, our data uncover a critical role for MuSK CRD in NMJ formation and function in adulthood.

Wnts proteins are known to be involved in muscle prepatterning early during NMJ formation (Wu et al., 2010). Moreover, in zebrafish, Wnt11 induced aneural AChRs clustering requires the CRD of Unpplugged/MuSKD(Jing et al., 2009). However, zebrafish lacking muscle prepatterning are able to form NMJ and are fully motile leaving open the question of the exact role of the prepatterning in NMJ functioning (Jing et al., 2009; Gordon et al., 2012). Here, we demonstrate that deletion of MuSK CRD in mammals severely impairs muscle prepatterning since the number of AChR clusters is drastically reduced (63%) and non innervated AChR clusters are strongly increased (30%) in E14 MuSKΔCRD embryos. Why deletion of MuSK CRD does not fully abolished muscle prepatterning remains unclear. Three hypothesis could explain this observation: first, it has been shown in vitro that deletion of MuSK CRD reduces but does not fully inhibit the binding activity of Wnt proteins to MuSK (Barik et al., 2014; Zhang et al., 2012). Thus, we cannot exclude that Wnts elicited muscle prepatterning requires other domains in MuSK. Second, Frizzled (Fzd) receptors are expressed in skeletal muscle cells and could mediate Wnt signaling to contribute to muscle prepatterning (Avilés et al., 2014; Strochlic et al., 2012). Finally, it has been suggested that MuSK and Lrp4 expression in early fused myofibers is sufficient to auto-activate MuSK and initiate muscle prepatterning (Burden et al., 2013; Kim and Burden, 2008). Wnts binding to MuSK CRD could therefore maintain or reinforce MuSK activation to amplify muscle prepatterning.

Our results demonstrate that MuSKΔCRD expression at the NMJ is increased by 70% in mutant compared to WT mice. In zebrafish, Wnt proteins have been shown to regulate the level of MuSK expression at the plasma membrane via activation of Wnt-induced MuSK endocytosis (Gordon et al., 2012). Therefore, deletion of MuSK CRD could disturb MuSK translocation from the plasma membrane to intracellular compartment leading to MuSK membrane accumulation and reduce Wnt/MuSK downstream signaling. Accordingly, we show that Wnt11-induced AChR clustering as well as β-catenin translocation to the nucleus are impaired in MuSKΔCRD primary myotubes suggesting that deletion of MuSK CRD alters downstream Wnt canonical signaling during NMJ formation.

With innervation, nerve terminals release agrin that binds to Lrp4 and subsequently increases MuSK phosphorylation further enhancing AChR clustering (Kim et al., 2008; Zhang et al., 2008, 2011). Among the key molecules involved in NMJ formation, MuSK, Lrp4, Rapsyn and Dok7 knock-out mice lack both aneural and agrin induced AChRs clusters (DeChiara et al., 1996; Gautam et al., 1999; Okada et al., 2006; Weatherbee et al., 2006). In contrast, although muscle prepatterning is severely affected in MuSKΔCRD embryos, our results show that at E18.5, upon innervation, NMJs are able to form but are abnormal with reduced AChR clusters number (30%), volume and density suggesting that (i) prepatterning is not indispensable but necessary for normal progression of NMJ differentiation, (ii) innervation only partially compensates early AChR clusters deficit. Since we show that MuSK CRD deletion does not affect agrin-induced AChR clustering in primary myotubes, Wnt proteins may bind to other receptors at the postsynaptic membrane including Fzd, thus activating signaling mechanisms leading to inhibition of agrin-induced AChR clustering. Consistent with this, it has been recently shown that Fzd9 is highly expressed in skeletal muscle when NMJs form and its overexpression in culture myotubes impairs agrin-induced AChR clustering (Avilés et al., 2014).

In MuSK null mutant mice, motor axons grow excessively throughout the muscle (DeChiara et al., 1996). Similarly, both during muscle prepatterning and later during NMJ formation, MuSKΔCRD motor axons overshoot AChR clusters and grow aberrantly all over the muscle. This result underlines the importance of the MuSK CRD in regulating a muscle retrograde stop signal for motor axons. In support of this hypothesis, studies of conditional invalidation or overexpression of muscle key components of Wnt canonical signaling including Lrp4 and β-catenin in vivo in mice suggest a role for Wnt canonical signaling to direct a retrograde signaling required for presynaptic differentiation (Li et al., 2008; Liu et al., 2012; Wu et al., 2012a, 2012b).

Intriguingly, despite severe NMJ formation defects, MuSKΔCRD mice are viable at birth and reach adulthood without any obvious abnormal phenotype during the first two weeks. In contrast, mice deficient for MuSK fail to form NMJs and die at birth due to respiratory failure (DeChiara et al., 1996). These data suggest that the remaining activity of MuSK deleted from its CRD is sufficient to prevent mutant mice from lethality. However, two weeks after birth, MuSKΔCRD mice start to develop CMS-like symptoms. Morphological analysis of adult MuSKΔCRD NMJs reveals abnormal endplates architecture, with an immature phenotype at P20 followed by a severe dismantlement at P60. This has been observed in all muscles analyzed including diaphragm, TA, soleus and EDL suggesting that MuSK CRD is necessary for all striated muscles to guarantee NMJ integrity. This abnormal NMJ phenotype is likely to be the consequence of early NMJ defects during development since NMJ defects similar to those observed in mutant embryos are detected in P5 MuSKΔCRD diaphragms. In this hypothesis, beside its role for synapse positioning early during NMJ formation, muscle prepatterning would also have a so far unsuspected role in NMJ functioning in adult mice. However, we cannot exclude a specific MuSK CRD-dependent role of Wnts during NMJ maintenance in adulthood. Further investigations are required to discriminate the role of MuSK CRD during NMJ formation in embryos and maintenance in adulthood.

NMJ fragmentation is often associated with muscle weakness as it has been described in CMS patients (Slater et al., 2006). Indeed, MuSKΔCRD adult mice develop fatigable muscle weakness highlighted by abnormal performance in the grip test assay, ex vivo isometric diaphragm contraction and fatigability measurement in response to nerve stimulation. Interestingly, one mutant mouse over five mice tested for isometric diaphragm contraction exhibits spontaneous twitches after a unique stimulation, a phenomenon often caused by muscle denervation (Heckmann and Ludin, 1982). In line with this observation, analysis of the NMJ innervation pattern in P90 MuSKΔCRD mice reveals the presence of non innervated AChR clusters suggesting a denervation-like process.

Electron microscopy analyses of adult MuSKΔCRD NMJs reveal defects in presynaptic vesicle density and postsynaptic folds structure associated with either few or highly disorganized junctional folds (JFs). AChR clusters and JFs are required for the genesis of an efficient endplate potential leading to the activation of the voltage-gated sodium channels concentrated in the depth of the JFs (Engel and Fumagalli, 1982; Marques et al., 2000). Thus, reduced presynaptic vesicle density, fragmented endplates as well as disorganized or fewer JFs are most probably responsible for the fatigable muscle weakness observed in adult MuSKΔCRD mice. Our investigation of the muscle pattern does not reveal major changes except mild muscle atrophy with reduced fiber size (data not shown). This muscle atrophy may participate to the muscle weakness but it may also result from defective NMJ maintenance since muscle activity is required for muscle trophicity (Schiaffino et al., 2007).

LiCl is currently used as a pharmacological reagent to treat bipolar, Parkinson's and Hungtinton's diseases (Klein and Melton, 1996; Schou, 2001; Chiu et al., 2011; Yong et al., 2011). Remarkably, LiCl treatment rescues beta-catenin signaling and improves the impaired NMJ defects in both MuSKΔCRD embryos and adult mice suggesting that the defects observed during NMJ formation and maintenance in MuSKΔCRD mice are in part due to inhibition of the Wnt canonical signaling pathway. However, given that Gsk3 is a pivotal kinase interacting with multiple signaling pathways including PI3K-PTEN-Akt-mTOR or Ras-Raf-MEK-ERK, we cannot rule out the possibility that LiCl regulates other signaling pathways involved in NMJ development (McCubrey et al., 2014). Interestingly, LiCl was shown to be efficient in the treatment of occulopharyngeal muscular dystrophy through activation of the Wnt canonical pathway and to improve skeletal muscle strength in mouse models of myotonic dystrophy (Abu-Baker et al., 2013; Jones et al., 2012). Our results provide the first evidence that LiCl or other Gsk3 inhibitors can also be used as therapeutic reagents or in complement to the treatment currently available of neuromuscular disorders associated to Wnt-MuSK deficiency.

### REFERENCES:

Throughout this application, various references describe the state of the art to which this invention pertains. The disclosures of these references are hereby incorporated by reference into the present disclosure.
Van Amerongen, R., and Nusse, R. (2009). Towards an integrated view of Wnt signaling in development. Dev. Camb. Engl. 136, 3205-3214.
Ben Ammar, A., Soltanzadeh, P., Bauché, S., Richard, P., Goillot, E., Herbst, R., Gaudon, K., Huzé, C., Schaeffer, L., Yamanashi, Y., et al. (2013). A mutation causes MuSK reduced sensitivity to agrin and congenital myasthenia. PloS One 8, e53826.
Arber, S., Burden, S.J., and Harris, A.J. (2002). Patterning of skeletal muscle. Curr. Opin. Neurobiol. 12, 100-103.
Avilés, E.C., Pinto, C., Hanna, P., Ojeda, J., Pérez, V., De Ferrari, G.V., Zamorano, P., Albistur, M., Sandoval, D., and Henríquez, J.P. (2014). Frizzled-9 impairs acetylcholine receptor clustering in skeletal muscle cells. Front. Cell. Neurosci. 8, 110.
Abu-Baker, A., Laganiere, J., Gaudet, R., Rochefort, D., Brais, B., Neri, C., Dion, P.A., and Rouleau, G.A. (2013). Lithium chloride attenuates cell death in oculopharyngeal muscular dystrophy by perturbing Wnt/β-catenin pathway. Cell Death Dis. 4, e821.
Barik, A., Zhang, B., Sohal, G.S., Xiong, W.-C., and Mei, L. (2014). Crosstalk between Agrin and Wnt signaling pathways in development of vertebrate neuromuscular junction. Dev. Neurobiol.
Berrih-Aknin, S., Frenkian-Cuvelier, M., and Eymard, B. (2014). Diagnostic and clinical classification of autoimmune myasthenia gravis. J. Autoimmun. 48-49, 143-148.
Budnik, V., and Salinas, P.C. (2011). Wnt signaling during synaptic development and plasticity. Curr. Opin. Neurobiol. 21, 151-159.
Cartaud, A., Strochlic, L., Guerra, M., Blanchard, B., Lambergeon, M., Krejci, E., Cartaud, J., and Legay, C. (2004). MuSK is required for anchoring acetylcholinesterase at the neuromuscular junction. J. Cell Biol. 165, 505-515.
Chevessier, F., Faraut, B., Ravel-Chapuis, A., Richard, P., Gaudon, K., Bauché, S., Prioleau, C., Herbst, R., Goillot, E., Ioos, C., et al. (2004). MUSK, a new target for mutations causing congenital myasthenic syndrome. Hum. Mol. Genet. 13, 3229-3240.
Chiu, C.-T., Liu, G., Leeds, P., and Chuang, D.-M. (2011). Combined treatment with the mood stabilizers lithium and valproate produces multiple beneficial effects in transgenic mouse models of Huntington's disease. Neuropsychopharmacol. Off. Publ. Am. Coll. Neuropsychopharmacol. 36, 2406-2421.
DeChiara, T.M., Bowen, D.C., Valenzuela, D.M., Simmons, M.V., Poueymirou, W.T., Thomas, S., Kinetz, E., Compton, D.L., Rojas, E., Park, J.S., et al. (1996). The receptor tyrosine kinase MuSK is required for neuromuscular junction formation in vivo. Cell 85, 501-512.
Dickins, E.M., and Salinas, P.C. (2013). Wnts in action: from synapse formation to synaptic maintenance. Front. Cell. Neurosci. 7, 162.
Engel, A.G., and Fumagalli, G. (1982). Mechanisms of acetylcholine receptor loss from the neuromuscular junction. Ciba Found. Symp. 197-224.
Eymard, B., Hantaï, D., and Estoumet, B. (2013). Congenital myasthenic syndromes. Handb. Clin. Neurol. 113, 1469-1480.
Gautam, M., DeChiara, T.M., Glass, D.J., Yancopoulos, G.D., and Sanes, J.R. (1999). Distinct phenotypes of mutant mice lacking agrin, MuSK, or rapsyn. Brain Res. Dev. Brain Res. 114, 171-178.
Gordon, L.R., Gribble, K.D., Syrett, C.M., and Granato, M. (2012). Initiation of synapse formation by Wnt-induced MuSK endocytosis. Dev. Camb. Engl. 139, 1023-1033.
Gould, T.D. (2006). Targeting glycogen synthase kinase-3 as an approach to develop novel mood-stabilising medications. Expert Opin. Ther. Targets 10, 377-392.
Guergueltcheva, V., Müller, J.S., Dusl, M., Senderek, J., Oldfors, A., Lindbergh, C., Maxwell, S., Colomer, J., Mallebrera, C.J., Nascimento, A., et al. (2011). Congenital myasthenic syndrome with tubular aggregates caused by GFPT1 mutations. J. Neurol.
Hantaï, D., Nicole, S., and Eymard, B. (2013). Congenital myasthenic syndromes: an update. Curr. Opin. Neurol. 26, 561-568.
Hesser, B.A., Henschel, O., and Witzemann, V. (2006). Synapse disassembly and formation of new synapses in postnatal muscle upon conditional inactivation of MuSK. Mol. Cell. Neurosci. 31, 470-480.
Hoch, W., McConville, J., Helms, S., Newsom-Davis, J., Melms, A., and Vincent, A. (2001). Auto-antibodies to the receptor tyrosine kinase MuSK in patients with myasthenia gravis without acetylcholine receptor antibodies. Nat. Med. 7, 365-368.
Jing, L., Lefebvre, J.L., Gordon, L.R., and Granato, M. (2009). Wnt signals organize synaptic prepattern and axon guidance through the zebrafish unplugged/MuSK receptor. Neuron 61, 721-733.
Jones, K., Wei, C., Iakova, P., Bugiardini, E., Schneider-Gold, C., Meola, G., Woodgett, J., Killian, J., Timchenko, N.A., and Timchenko, L.T. (2012). GSK3β mediates muscle pathology in myotonic dystrophy. J. Clin. Invest. 122, 4461-4472.
Kim, N., and Burden, S.J. (2008). MuSK controls where motor axons grow and form synapses. Nat. Neurosci. 11, 19-27.
Kim, N., Stiegler, A.L., Cameron, T.O., Hallock, P.T., Gomez, A.M., Huang, J.H., Hubbard, S.R., Dustin, M.L., and Burden, S.J. (2008). Lrp4 is a receptor for Agrin and forms a complex with MuSK. Cell 135, 334-342.
Klein, P.S., and Melton, D.A. (1996). A molecular mechanism for the effect of lithium on development. Proc. Natl. Acad. Sci. U. S. A. 93, 8455-8459.
Kummer, T.T., Misgeld, T., and Sanes, J.R. (2006). Assembly of the postsynaptic membrane at the neuromuscular junction: paradigm lost. Curr. Opin. Neurobiol. 16, 74-82.
Li, X.-M., Dong, X.-P., Luo, S.-W., Zhang, B., Lee, D.-H., Ting, A.K.L., Neiswender, H., Kim, C.-H., Carpenter-Hyland, E., Gao, T.-M., et al. (2008). Retrograde regulation of motoneuron differentiation by muscle beta-catenin. Nat. Neurosci. 11, 262-268.
Lin, S., Landmann, L., Ruegg, M.A., and Brenner, H.R. (2008). The role of nerve-versus muscle-derived factors in mammalian neuromuscular junction formation. J. Neurosci. Off. J. Soc. Neurosci. 28, 3333-3340.
Lin, W., Burgess, R.W., Dominguez, B., Pfaff, S.L., Sanes, J.R., and Lee, K.F. (2001). Distinct roles of nerve and muscle in postsynaptic differentiation of the neuromuscular synapse. Nature 410, 1057-1064.
Liu, Y., Sugiura, Y., Wu, F., Mi, W., Taketo, M.M., Cannon, S., Carroll, T., and Lin, W. (2012). β-Catenin stabilization in skeletal muscles, but not in motor neurons, leads to aberrant motor innervation of the muscle during neuromuscular development in mice. Dev. Biol. 366, 255-267.
Makoukji, J., Belle, M., Meffre, D., Stassart, R., Grenier, J., Shackleford, G., Fledrich, R., Fonte, C., Branchu, J., Goulard, M., et al. (2012a). Lithium enhances remyelination of peripheral nerves. Proc. Natl. Acad. Sci. U. S. A. 109, 3973-3978.
Makoukji, J., Belle, M., Meffre, D., Stassart, R., Grenier, J., Shackleford, G., Fledrich, R., Fonte, C., Branchu, J., Goulard, M., et al. (2012b). Lithium enhances remyelination of peripheral nerves. Proc. Natl. Acad. Sci. U. S. A. 109, 3973-3978.
Von Maltzahn, J., Chang, N.C., Bentzinger, C.F., and Rudnicki, M.A. (2012). Wnt signaling in myogenesis. Trends Cell Biol. 22, 602-609.
Marques, M.J., Conchello, J.A., and Lichtman, J.W. (2000). From plaque to pretzel: fold formation and acetylcholine receptor loss at the developing neuromuscular junction. J. Neurosci. Off. J. Soc. Neurosci. 20, 3663-3675.
Maselli, R.A., Arredondo, J., Cagney, O., Ng, J.J., Anderson, J.A., Williams, C., Gerke, B.J., Soliven, B., and Wollmann, R.L. (2010). Mutations in MUSK causing congenital myasthenic syndrome impair MuSK-Dok-7 interaction. Hum. Mol. Genet. 19, 2370-2379.
Masiakowski, P., and Yancopoulos, G.D. (1998). The Wnt receptor CRD domain is also found in MuSK and related orphan receptor tyrosine kinases. Curr. Biol. CB 8, R407.
McCubrey, J.A., Steelman, L.S., Bertrand, F.E., Davis, N.M., Sokolosky, M., Abrams, S.L., Montalto, G., D'Assoro, A.B., Libra, M., Nicoletti, F., et al. (2014). GSK-3 as potential target for therapeutic intervention in cancer. Oncotarget 5, 2881-2911.
Mihaylova, V., Salih, M.A.M., Mukhtar, M.M., Abuzeid, H.A., El-Sadig, S.M., von der Hagen, M., Huebner, A., Nümberg, G., Abicht, A., Müller, J.S., et al. (2009). Refinement of the clinical phenotype in musk-related congenital myasthenic syndromes. Neurology 73, 1926-1928.
Morgan-Hughes, J.A. (1998). Tubular aggregates in skeletal muscle: their functional significance and mechanisms of pathogenesis. Curr. Opin. Neurol. 11, 439-442.
Okada, K., Inoue, A., Okada, M., Murata, Y., Kakuta, S., Jigami, T., Kubo, S., Shiraishi, H., Eguchi, K., Motomura, M., et al. (2006). The muscle protein Dok-7 is essential for neuromuscular synaptogenesis. Science 312, 1802-1805.
Punga, A.R., Maj, M., Lin, S., Meinen, S., and Rüegg, M.A. (2011). MuSK levels differ between adult skeletal muscles and influence postsynaptic plasticity. Eur. J. Neurosci. 33, 890-898.
Salinas, P.C. (2012). Wnt signaling in the vertebrate central nervous system: from axon guidance to synaptic function. Cold Spring Harb. Perspect. Biol. 4.
Sanes, J.R., and Lichtman, J.W. (2001). Induction, assembly, maturation and maintenance of a postsynaptic apparatus. Nat. Rev. Neurosci. 2, 791-805.
Schou, M. (2001). Lithium treatment at 52. J. Affect. Disord. 67, 21-32.
Slater, C.R., Fawcett, P.R.W., Walls, T.J., Lyons, P.R., Bailey, S.J., Beeson, D., Young, C., and Gardner-Medwin, D. (2006). Pre- and post-synaptic abnormalities associated with impaired neuromuscular transmission in a group of patients with "limb-girdle myasthenia."Brain J. Neurol. 129, 2061-2076.
Speese, S.D., and Budnik, V. (2007). Wnts: up-and-coming at the synapse. Trends Neurosci. 30, 268-275.
Stambolic, V., Ruel, L., and Woodgett, J.R. (1996). Lithium inhibits glycogen synthase kinase-3 activity and mimics wingless signalling in intact cells. Curr. Biol. CB 6, 1664-1668.
Stiegler, A.L., Burden, S.J., and Hubbard, S.R. (2009). Crystal structure of the frizzled-like cysteine-rich domain of the receptor tyrosine kinase MuSK. J. Mol. Biol. 393, 1-9.
Strochlic, L., Falk, J., Goillot, E., Sigoillot, S., Bourgeois, F., Delers, P., Rouvière, J., Swain, A., Castellani, V., Schaeffer, L., et al. (2012). Wnt4 Participates in the Formation of Vertebrate Neuromuscular Junction. PLoS ONE 7, e29976.
Takamori, M. (2012). Structure of the neuromuscular junction: function and cooperative mechanisms in the synapse. Ann. N. Y. Acad. Sci. 1274, 14-23.
Takamori, M., Nakamura, T., and Motomura, M. (2013). Antibodies against Wnt receptor of muscle-specific tyrosine kinase in myasthenia gravis. J. Neuroimmunol. 254, 183-186.
Valenzuela, D.M., Stitt, T.N., DiStefano, P.S., Rojas, E., Mattsson, K., Compton, D.L., Nuñez, L., Park, J.S., Stark, J.L., and Gies, D.R. (1995). Receptor tyrosine kinase specific for the skeletal muscle lineage: expression in embryonic muscle, at the neuromuscular junction, and after injury. Neuron 15, 573-584.
Vincent, A., and Leite, M.I. (2005). Neuromuscular junction autoimmune disease: muscle specific kinase antibodies and treatments for myasthenia gravis. Curr. Opin. Neurol. 18, 519-525.
Wada, A. (2009). Lithium and neuropsychiatric therapeutics: neuroplasticity via glycogen synthase kinase-3beta, beta-catenin, and neurotrophin cascades. J. Pharmacol. Sci. 110, 14-28.
Weatherbee, S.D., Anderson, K.V., and Niswander, L.A. (2006). LDL-receptor-related protein 4 is crucial for formation of the neuromuscular junction. Dev. Camb. Engl. 133, 4993-5000.
Willert, K., and Nusse, R. (2012). Wnt proteins. Cold Spring Harb. Perspect. Biol. 4, a007864.
Wu, H., Xiong, W.C., and Mei, L. (2010). To build a synapse: signaling pathways in neuromuscular junction assembly. Dev. Camb. Engl. 137, 1017-1033.
Wu, H., Lu, Y., Barik, A., Joseph, A., Taketo, M.M., Xiong, W.-C., and Mei, L. (2012a). β-Catenin gain of function in muscles impairs neuromuscular junction formation. Dev. Camb. Engl. 139, 2392-2404.
Wu, H., Lu, Y., Shen, C., Patel, N., Gan, L., Xiong, W.C., and Mei, L. (2012b). Distinct roles of muscle and motoneuron LRP4 in neuromuscular junction formation. Neuron 75, 94-107.
Yang, X., Arber, S., William, C., Li, L., Tanabe, Y., Jessell, T.M., Birchmeier, C., and Burden, S.J. (2001). Patterning of muscle acetylcholine receptor gene expression in the absence of motor innervation. Neuron 30, 399-410.
Yong, Y., Ding, H., Fan, Z., Luo, J., and Ke, Z.-J. (2011). Lithium fails to protect dopaminergic neurons in the 6-OHDA model of Parkinson's disease. Neurochem. Res. 36, 367-374.
Zhang, B., Luo, S., Wang, Q., Suzuki, T., Xiong, W.C., and Mei, L. (2008). LRP4 serves as a coreceptor of agrin. Neuron 60, 285-297.
Zhang, B., Liang, C., Bates, R., Yin, Y., Xiong, W.-C., and Mei, L. (2012). Wnt proteins regulate acetylcholine receptor clustering in muscle cells. Mol. Brain 5, 7.
Zhang, W., Coldefy, A.-S., Hubbard, S.R., and Burden, S.J. (2011). Agrin binds to the N-terminal region of Lrp4 protein and stimulates association between Lrp4 and the first immunoglobulin-like domain in muscle-specific kinase (MuSK). J. Biol. Chem. 286, 40624-40630.

## Claims

1. An inhibitor of glycogen synthase kinase 3 (GSK3) for use in treating a neuromuscular junction-related disease in a subject, wherein said disease is selected from the group consisting of myasthenia gravis, Lambert-Eaton syndrome, Miller Fischer syndrome, congenital myasthenic syndromes, botulism, organophosphate poisoning and poisoning with any other toxin that compromises the neuromuscular junction, wherein said inhibitor of GSK3 is zentylor.

2. The inhibitor of GSK3 for use according to claim 1, wherein said disease is myasthenia gravis.

3. The inhibitor of GSK3 for use according to claim 1, wherein said disease is congenital myasthenic syndrome.

4. The inhibitor of GSK3 for use according to claim 1, wherein the subject is human.
